(19) 

Europäisches
Patentamt
European
Patent Office
Office européen
des brevets

(11) **EP 4 212 864 A1**

(12) **EUROPEAN PATENT APPLICATION**
published in accordance with Art. 153(4) EPC

(43) Date of publication:
**19.07.2023 Bulletin 2023/29**

(21) Application number: **20953249.8**

(22) Date of filing: **09.09.2020**

(51) International Patent Classification (IPC):
*G01N 27/62* (2021.01)        *G01N 33/50* (2006.01)

(52) Cooperative Patent Classification (CPC):
**G01N 27/62; G01N 33/50**

(86) International application number:
**PCT/JP2020/034198**

(87) International publication number:
**WO 2022/054183 (17.03.2022 Gazette 2022/11)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **HITACHI HIGH-TECH CORPORATION
Tokyo 105-6409 (JP)**

(72) Inventors:
- **SAKAIRI Minoru
  Tokyo 100-8280 (JP)**
- **ABE Mayumi
  Tokyo 100-8280 (JP)**
- **ISHIGAKI Takashi
  Tokyo 100-8280 (JP)**

(74) Representative: **MERH-IP Matias Erny Reichl
Hoffmann
Patentanwälte PartG mbB
Paul-Heyse-Strasse 29
80336 München (DE)**

(54) **CANCER TEST METHOD USING METABOLITE LIST**

(57)    The present invention provides a cancer test method and a cancer test system for evaluating cancer in a subject. Specifically, the present invention provides a method for testing cancer in a subject, the method including: preparing a database that has stored a marker panel on which information of multiple cancer markers with respect to multiple healthy subjects and cancer patients is registered, the database including discrimination information that classifies a measured value of each cancer marker, with a mean value of the healthy subjects $\pm$ X $\times$ standard deviation (wherein X is an arbitrary numerical value) and/or the mean value of the healthy subjects $\pm$ the standard deviation as a reference range, into any of three groups: within the reference range, higher than the reference range, and lower than the reference range; analyzing, with respect to measured values of one or more cancer markers of the subject, a correlation with the discrimination information in the database; and evaluating cancer in the subject on the basis of a result of the analysis.

**EP 4 212 864 A1**

**Description**

Technical Field

The present invention relates to a cancer test method and a cancer test system for evaluating cancer in a subject.

Background Art

In a conventional cancer test method using a cancer marker, first, candidate markers are comprehensively analyzed, and, from detected candidate markers, a promising candidate marker is extracted by statistical analysis and machine learning, and then a prediction formula for discriminating between healthy subjects and cancer patients is created by multivariate analysis. Next, the test is performed by a process of substituting a measured value of a marker in a subject into the prediction formula and determining a risk of cancer from an obtained predictive value.

The present inventors have also comprehensively analyzed urinary metabolites as cancer markers by means of a liquid chromatograph/mass spectrometer (LC/MS), and have multiplied the intensities of multiple urinary tumor markers by a given coefficient, and then, in accordance with a prediction formula: a predictive value = $\Sigma$ ($\alpha_n \times I_n$) + $\beta$ [$\alpha_n$: coefficient, $\beta$: constant, $I_n$: intensity of urinary tumor marker], have determined that a risk of cancer is high if the predictive value is equal to or greater than 0 and that the risk is low if the predictive value is smaller than 0 (for example, PTLS 1 and 2).

Citation List

Patent Literatures

PTL 1: JP 2019-105456 A
PTL 2: JP 2020-079729 A
PTL 3: WO 2007/076439 A
PTL 4: WO 2011/119772 A
PTL 5: US 2009/0004687 A1

Summary of Invention

Technical Problem

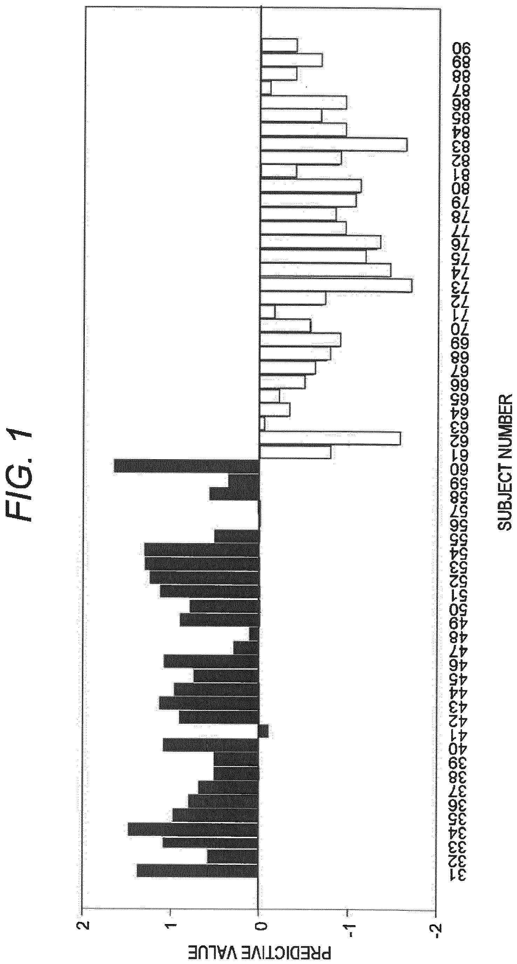

However, in a case of using such a prediction formula, depending on a combination of a coefficient and an intensity of a cancer marker, a predictive value may be close to 0, which makes the determination difficult in some cases. FIG. 1 shows a specific example of distinction based on such a prediction formula. FIG. 1 is a result of cancer risk determination made using 15 types of urinary tumor markers in colorectal cancer cases (30 cases of colorectal cancer patients and 30 cases of healthy subjects). In an area of the cancer patients, predictive values of about four cases are numerical values close to 0; also in an area of the healthy subjects, one case has a value close to 0. This predictive value method does not allow for further consideration, which proves that in some cases, it is difficult to precisely determine the cancer risk from only a predictive value.

Meanwhile, the idea of a metabolite list as a marker set already exists. For example, according to PTLS 3 to 5, in a cancer test using metabolites as markers, a metabolite list including multiple metabolites is created. However, in the conventional techniques, there are neither reports nor suggestions about: at the time of evaluation of cancer risk, with respect to individual metabolite markers, setting a reference range of healthy subjects that is training data and classifying the metabolite markers into three groups; performing similar calculation on the same multiple metabolites in test data as well; or analyzing which pattern in the metabolite list created on the basis of the training data a metabolite pattern of the test data belongs to.

Accordingly, an object of the present invention is to provide a cancer test method and system for evaluating cancer with higher accuracy and precision.

Solution to Problem

The present inventors have found that in a cancer test method using cancer markers, evaluation of cancer can be made more precisely by analyzing measured values of the cancer markers by classifying them into three groups:

within a reference range of healthy subjects, higher than the reference range, and lower than the reference range. Furthermore, the present inventors have found that the evaluation of cancer can be made with ease and precision by displaying the cancer markers to be visually distinguishable among the three groups and/or by assigning column values to the three groups and substituting the column values into an evaluation function indicating an estimate of the amount of variation of a marker and/or a distance function indicating a degree of similarity of a marker pattern.

The present invention encompasses, for example, the following.

[1] A method for testing cancer in a subject, the method including:

preparing a database that has stored a marker panel on which information of multiple cancer markers with respect to multiple healthy subjects and cancer patients is registered, the database including discrimination information that classifies a measured value of each cancer marker, with a mean value of the healthy subjects $\pm$ X $\times$ standard deviation (wherein X is an arbitrary numerical value) and/or the mean value of the healthy subjects $\pm$ the standard deviation as a reference range, into any of three groups: within the reference range, higher than the reference range, and lower than the reference range;
analyzing, with respect to measured values of one or more cancer markers of the subject, a correlation with the discrimination information in the database; and
evaluating cancer in the subject on the basis of a result of the analyzing.

[2] The method according to [1], in which a marker panel of the subject is created on the basis of the measured values of the cancer markers of the subject.
[3] The method according to [1] or [2], in which, in the marker panel, columns of the cancer markers are displayed to be visually distinguishable according to three groups of the discrimination information.
[4] The method according to any one of [1] to [3], in which, in the marker panel, the cancer markers are shown in order of importance calculated by machine learning.
[5] The method according to any of [1] to [4], in which in the database, the reference range includes a reference range of the mean value of the healthy subjects $\pm$ X $\times$ the standard deviation (wherein X is 2) and a reference range of the mean value of the healthy subjects $\pm$ the standard deviation, and a measured value of each cancer marker has different discrimination information for the two reference ranges.
[6] The method according to any of [1] to [5], in which, in accordance with three groups of the discrimination information, the measured values of the cancer markers are assigned a column value of 0 if it is within the reference range, a column value of + 1 if it is higher than the reference range, or a column value of - 1 if it is lower than the reference range.
[7] The method according to [6], in which

the analyzing the correlation with the discrimination information is performed on the basis of a value of an evaluation function represented by Formula I:

$$\text{Evaluation function} = \sum_{n=1}^{m} \pm g_n \times \sqrt{(D_n^2)} \quad (I)$$

(wherein,
$g_n$ denotes a relative value of importance calculated by machine learning, and a sign of $g_n$ is + if a measured value of a cancer marker of the subject has the same column value as that of the cancer patients and - if it has a different column value; and
$D_n$ denotes a column value of + 1, 0, or - 1).

[8] The method according to [7], in which the analyzing the correlation with the discrimination information is performed by calculating an evaluation function on the basis of the discrimination information with the mean value of the healthy subjects $\pm$ X $\times$ the standard deviation (wherein X is 2) as the reference range, and then calculating an evaluation function on the basis of the discrimination information with the mean value of the healthy subjects $\pm$ the standard deviation as the reference range.
[9] The method according to any of [1] to [8], further including: with respect to the measured values of the one or more cancer markers of the subject, calculating differences from a pattern of the discrimination information of the cancer patients and a pattern of the discrimination information of the healthy subjects in the database; and analyzing which of the patterns of the cancer patients and the healthy subjects the measured values of the subject is close to.
[10] The method according to [9], in which the calculating the differences from the patterns is performed with a distance function represented by Formula II:

$$\text{Distance function} = \sqrt{\sum_{n=1}^{m} g_n^2 \times (D_n - T_n)^2} \quad \text{(II)}$$

(wherein,

$g_n$ denotes a relative value of importance calculated by machine learning;
$D_n$ denotes a row vector represented by a column value of + 1, 0, or - 1 with respect to each cancer marker of the cancer patients or the healthy subjects in the database; and
$T_n$ denotes a row vector represented by a column value of + 1, 0, or - 1 with respect to each cancer marker in the subject).

[11] The method according to any of [1] to [10], in which the database includes a marker panel on which information of multiple cancer markers with respect to a cancer patient in a specific stage or with a specific degree of severity is registered.

[12] The method according to any of [1] to [11], in which the cancer markers include 3 or more, 5 or more, 10 or more, or 20 or more cancer markers.

[13] The method according to any of [1] to [12], in which the cancer markers are urinary metabolites, and the marker panel on which the information of the cancer markers is registered is a metabolite list on which information of the ion intensities of metabolites obtained by liquid chromatography mass spectrometry (LC/MS) is registered.

[14] The method according to any of [1] to [13], in which the database includes different databases according to the types of cancers.

[15] The method according to any of [1] to [14], in which the evaluating cancer includes determination of cancer in the subject, prediction of a risk of cancer in the subject, determination of the stage or severity of cancer in the subject, prognostication of cancer in the subject, monitoring of cancer in the subject, monitoring of efficacy in the treatment of cancer present in the subject, or aid in diagnosis of cancer.

[16] A system for testing cancer including:

a storage unit including a database that has stored a marker panel on which information of multiple cancer markers with respect to multiple healthy subjects and cancer patients is registered, the database including discrimination information that classifies a measured value of each cancer marker, with a mean value of the healthy subjects $\pm$ X $\times$ standard deviation (wherein X is an arbitrary numerical value) and/or the mean value of the healthy subjects $\pm$ the standard deviation as a reference range, into any of three groups: within the reference range, higher than the reference range, and lower than the reference range;
an input unit that is configured to receive inputs of measured values of one or more cancer markers of a subject;
an analysis unit that is configured to analyze, with respect to the measured values of the one or more cancer markers of the subject from the input unit, a correlation with the discrimination information in the database; and
an evaluation unit that is configured to evaluate cancer in the subject on the basis of an analysis result obtained by the analysis unit.

[17] The system according to [16], in which the system is configured to implement the method according to [1].

Advantageous Effects of Invention

According to the present invention, a cancer test method and system for accurately and precisely evaluating cancer are provided. The method and system of the present invention are excellent in sensitivity and specificity, and therefore reduces false positives and false negatives and helps in precise diagnosis. Therefore, the present invention may be useful in the fields of cancer diagnosis, examination, treatment evaluation, and the like.

Brief Description of Drawings

[FIG. 1] FIG. 1 is a graph showing a result of distinction between colorectal cancer patients and healthy subjects based on a conventional prediction formula by means of 15 caner markers.
[FIG. 2] FIG. 2 is a schematic diagram of a method for dividing data of cancer markers into three groups and analyzing the cancer markers using a marker panel.
[FIG. 3] FIG. 3 shows a flow of a process for analyzing the importance of a marker (a metabolite).

[FIG. 4] FIG. 4 is a graph showing an example of placement of markers (metabolites) based on the importance obtained by a random forest (RF) method, a type of machine learning, as an index.

[FIG. 5] FIG. 5 is a schematic diagram illustrating an evaluation function of a cancer test method according to the present invention.

[FIG. 6] FIG. 6 is a schematic diagram illustrating a distance function of the cancer test method according to the present invention.

[FIG. 7] FIG. 7 shows a flow of determination algorithm 1 that is an embodiment of the cancer test method according to the present invention.

[FIG. 8] FIG. 8 shows a flow of determination algorithm 2 that is another embodiment of the cancer test method according to the present invention.

[FIG. 9] FIG. 9 shows the types of 17 types of urinary tumor markers (metabolites) used for evaluation of colorectal cancer in Example 1.

[FIG. 10] FIG. 10 shows an example of a metabolite list created in the evaluation of colorectal cancer in Example 1 (evaluation by an evaluation function in $2\sigma$ mode).

[FIG. 11] FIG. 11 shows an evaluation function plot in $2\sigma$ mode created in the evaluation of colorectal cancer in Example 1.

[FIG. 12] FIG. 12 shows a flow of analysis by the determination algorithm 1 based on results in $2\sigma$ mode and $1\sigma$ mode in the evaluation of colorectal cancer in Example 1.

[FIG. 13] FIG. 13 shows an evaluation function plot in $2\sigma$ mode (on the left) and an evaluation function plot in $1\sigma$ mode (on the right) that were created in the evaluation of colorectal cancer in Example 1.

[FIG. 14] FIG. 14 is a table on which results of determinations by the determination algorithm 1 in the evaluation of colorectal cancer in Example 1 have been summarized.

[FIG. 15] FIG. 15 shows a flow of analysis by determination algorithm 2 based on the evaluation function and a distance function in evaluation of colorectal cancer in Example 2.

[FIG. 16] FIG. 16 shows a flow of analysis by the determination algorithm 2 based on the evaluation function and the distance function in the evaluation of colorectal cancer in Example 2.

[FIG. 17] FIG. 17 is a table on which results of determinations by the determination algorithm 2 in the evaluation of colorectal cancer in Example 2 have been summarized.

[FIG. 18] FIG. 18 shows an example of a metabolite list of a cancer patient with a specific depth of invasion (T) created in evaluation of colorectal cancer in Example 3.

[FIG. 19] FIG. 19 shows a plot of an evaluation function with respect to the depth of invasion (T) in the evaluation of colorectal cancer in Example 3.

[FIG. 20] FIG. 20 shows the types of 15 urinary tumor markers (metabolites) used for evaluation of breast cancer in Example 4.

[FIG. 21] FIG. 21 shows an example of a metabolite list created in the evaluation of breast cancer in Example 4 (evaluation by an evaluation function in $2\sigma$ mode).

[FIG. 22] FIG. 22 shows an evaluation function plot in $2\sigma$ mode created in the evaluation of breast cancer in Example 4.

[FIG. 23] FIG. 23 shows a flow of analysis by the determination algorithm 1 based on results in $2\sigma$ mode and $1\sigma$ mode in the evaluation of breast cancer in Example 4.

[FIG. 24] FIG. 24 shows the evaluation function plot in $2\sigma$ mode (on the left) and an evaluation function plot in $1\sigma$ mode (on the right) that were created in the evaluation of breast cancer in Example 4, in which "CANCER" denotes a cancer patient number, and "HEALTHY" denotes a healthy subject number.

[FIG. 25] FIG. 25 is a table on which results of determinations by the determination algorithm 1 in the evaluation of breast cancer in Example 4 have been summarized, in which "CANCER" denotes a cancer patient number, and "HEALTHY" denotes a healthy subject number.

[FIG. 26] FIG. 26 shows a flow of analysis by the determination algorithm 2 based on the evaluation function and the distance function in the evaluation of breast cancer in Example 4.

[FIG. 27] FIG. 27 is a table on which results (cancer patients) of determinations by the determination algorithm 2 in the evaluation of breast cancer in Example 4 have been summarized.

[FIG. 28] FIG. 28 is a table on which results (healthy subjects) of determinations by the determination algorithm 2 in the evaluation of breast cancer in Example 4 have been summarized.

[FIG. 29] FIG. 29 shows a configuration example of a system according to the present invention.

Description of Embodiments

In the present specification, "$\sigma$", the symbol denoting standard deviation, all represents: $\hat{\sigma}$, that is, sigma-hat (a population estimate).

The present invention provides a cancer test method and a cancer test system for evaluating cancer in a subject.

According to the present invention, evaluation of cancer may include determination of cancer in a subject, prediction of a risk of cancer in a subject, determination of the stage or severity of cancer in a subject, prognostication of cancer in a subject, monitoring of cancer in a subject, monitoring of efficacy in treatment of cancer present in a subject, or aid in diagnosis of cancer.

An aspect of the present invention is to provide a method for testing cancer in a subject, the method including:

preparing a database that has stored a marker panel on which information of multiple cancer markers with respect to multiple healthy subjects and cancer patients is registered, the database including discrimination information that classifies a measured value of each cancer marker, with a mean value of the healthy subjects ± X × standard deviation (wherein X is an arbitrary numerical value) and/or the mean value of the healthy subjects ± the standard deviation as a reference range, into any of three groups: within the reference range, higher than the reference range, and lower than the reference range;

with respect to measured values of one or more cancer markers of a subject, analyzing a correlation with the discrimination information in the database; and

evaluating cancer in the subject on the basis of a result of the analyzing.

In the cancer test method according to the present invention, in accordance with a measured value of each cancer marker, the multiple cancer markers may be classified into three groups: within a specific reference range, higher than the reference range, and lower than the reference range.

The cancer markers are not particularly limited as long as they are markers that show association with cancer. For example, a specific protein, a miRNA, and a metabolite are known as a cancer marker, and the cancer marker can be either a marker in blood or in urine. Multiple cancer markers may be preferable, and may include, for example, 2 or more, 3 or more, 4 or more, 5 or more, 10 or more, 15 or more, 20 or more, 25 or more, 30 or more, 40 or more, 50 or more, or more cancer markers. There is not particularly an upper limit to the number of the cancer markers; however, the upper limit can be, for example, up to 500, up to 400, or up to 300. The cancer markers may preferably be multiple markers that can be measured at the same time. In a preferred embodiment, the cancer markers may be urinary metabolites. A "measured value" of a cancer marker may differ depending on the type of marker used, which can be understood by those skilled in the art.

First, from data of measured values of cancer markers of multiple healthy subjects, a mean value of the healthy subjects ± X × standard deviation (wherein X is an arbitrary numerical value) and/or the mean value of the healthy subjects ± the standard deviation may be set as a reference range. In one embodiment, in a case where X is 2, a reference range of 95%, i.e., $2\sigma$ mode (a range sandwiched between (the mean value - 2 × the standard deviation) and (the mean value + 2 × the standard deviation)) and/or a reference range of 68% that is $1\sigma$ mode (a range sandwiched between (the mean value - the standard deviation) and (the mean value + the standard deviation)) may be set.

With respect to measured values of cancer markers of multiple cancer patients and multiple healthy subjects, discrimination information that classifies the cancer markers into three groups: within the reference range, higher than the reference range, and lower than the reference range may be obtained on the basis of the reference range. In a preferred embodiment, the measured value of each cancer marker has discrimination information that differs between the two reference ranges: the reference range based on the mean value of the healthy subjects ± X × the standard deviation (wherein X is 2) and the reference range based on the mean value of the healthy subjects ± the standard deviation. In the database, the measured values of the cancer markers may be stored together with the discrimination information. Therefore, the database may include, as information of the cancer markers, at least the types of the cancer markers, the measured values of the cancer markers, and the discrimination information.

The database may include a marker panel on which information of multiple cancer markers with respect to cancer patients in a specific stage or with a specific degree of severity (for example, such as a specific depth of invasion T, a specific grade, or the presence or absence of metastasis or recurrence) or cancer patients before or after treatment may be registered. Furthermore, the database may include different databases according to the types of cancers. The types of cancers may not be particularly limited, and, may include, for example, colorectal cancer, breast cancer, pediatric cancer, etc.

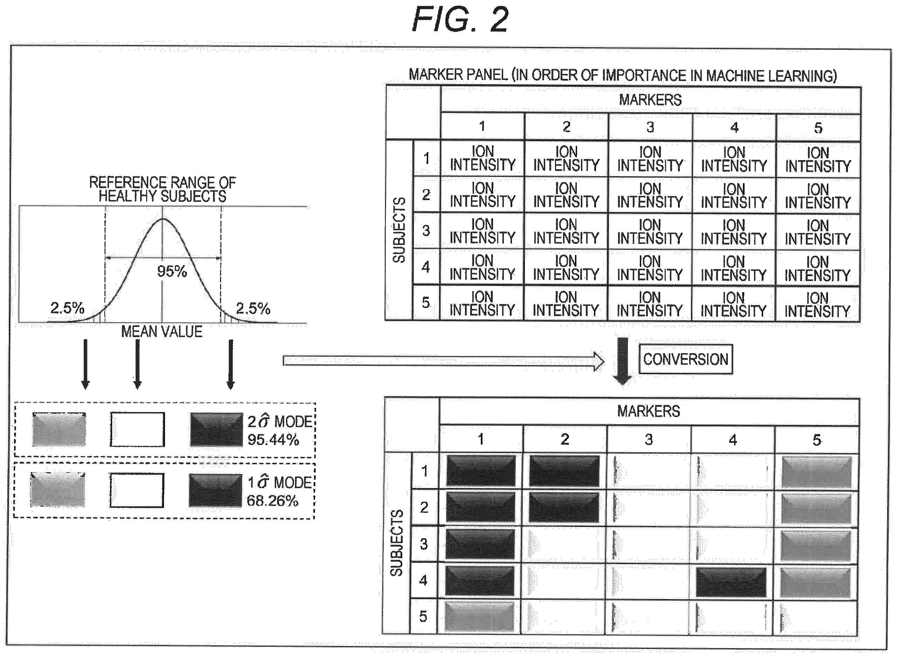

The information of the cancer markers may be stored as a marker panel in the database. A "metabolite list" means a set of multiple markers for each cancer in cancer patients and healthy subjects, and a table in which measured values and/or discrimination information are arranged is referred to as a marker panel. For example, there is a tabular marker panel like one shown in FIG. 2. In a preferred embodiment, the marker panel with the information of the cancer markers registered thereon may be a metabolite list with information of the ion intensities of metabolites obtained by means of a liquid chromatography mass spectrometry (LC/MS) registered thereon.

In the marker panel, preferably, columns of the cancer markers may be displayed to be visually distinguishable according to three groups of discrimination information. For example, the columns can be displayed to be distinguishable by shades of color, by different colors, or by lighting. For example, as shown in the lower right part of FIG. 2, the three groups of discrimination information can be displayed to be distinguishable by shades of color (black, white, and gray).

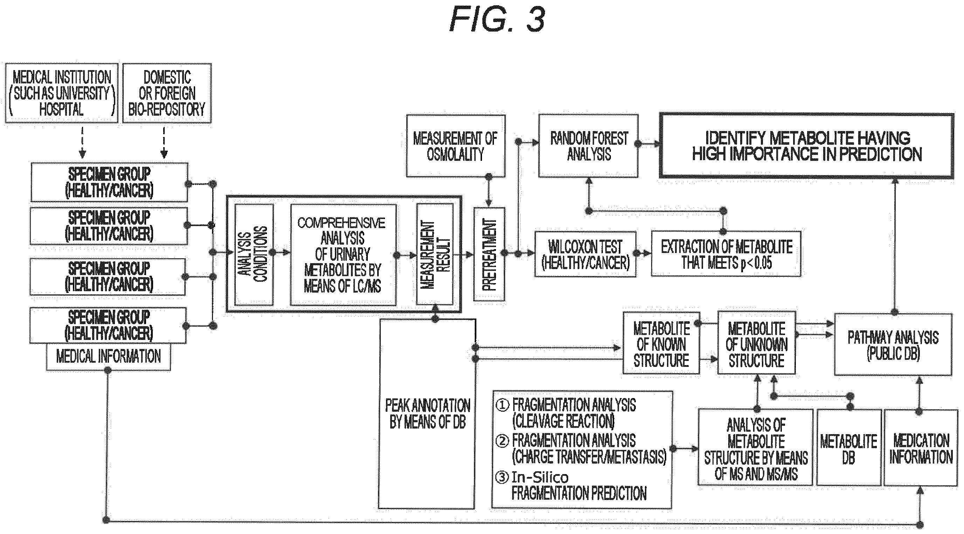

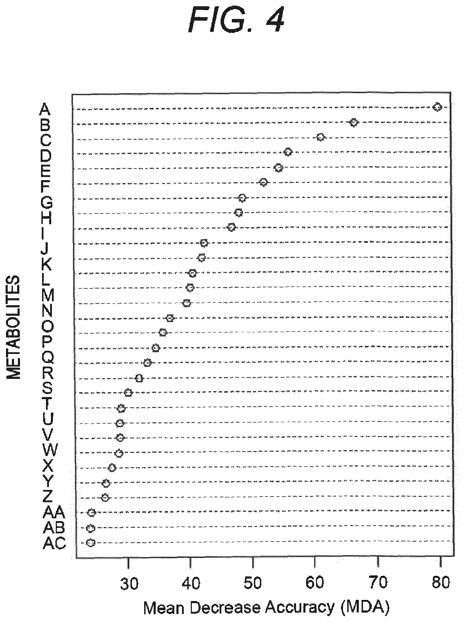

When the marker panel is created, preferably, the cancer markers may be shown in order of importance calculated by machine learning. For example, in FIG. 2, the markers are displayed to be arranged in order of importance obtained by a random forest (RF) method, which is a type of machine learning. An analysis process leading up to this machine learning (for example, the RF method) is the same as in the case of the predictive value method using metabolites as markers. For reference, FIG. 3 shows an analysis process of a conventional predictive value method. Basically, about 2000 types of metabolites of high importance extracted by comprehensive analysis may be extracted in stages through data pretreatment, statistical analysis, etc., and an index of the importance is obtained by a random forest (RF) method. FIG. 4 shows an example of a result of analysis of an index of the importance (mean decrease accuracy: MDA) obtained by the random forest (RF) method.

Measured values of one or more cancer markers of a subject may also be classified into three groups: within a reference range, higher than the reference range, and lower than the reference range. In a preferred embodiment, a marker panel of the subject may be created on the basis of the measured values of the cancer markers of the subject.

The marker panel including the discrimination information in the database obtained in this way may be compared with the marker panel of the cancer markers of the subject, and a correlation of the measured values of the cancer markers of the subject with the discrimination information in the database may be analyzed. This analysis can be made by a method publicly known in this technical field. In one embodiment, the three groups of discrimination information may be assigned numerical values, and, taking into account the importance calculated by machine learning, the correlation can be analyzed by a function. A specific example of that is described below.

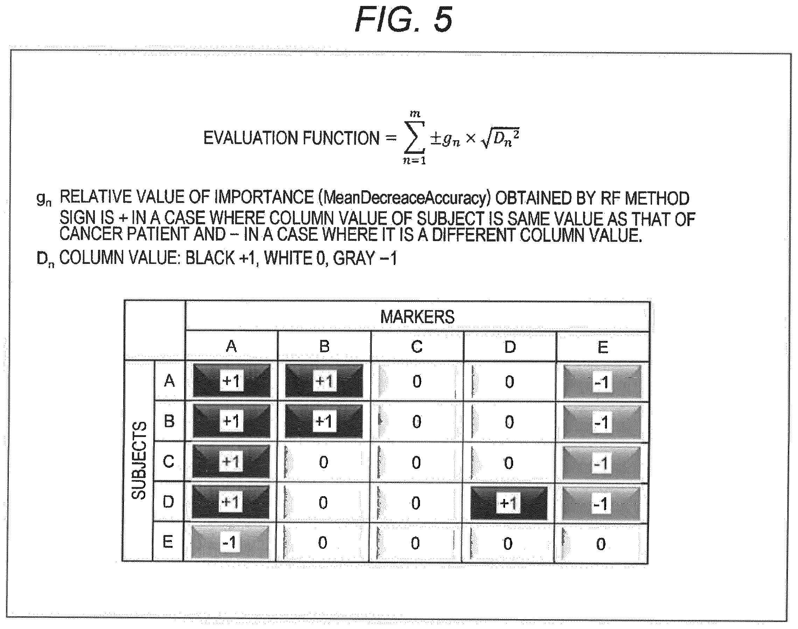

For the purpose of analysis of the correlation with the discrimination information, it may be preferable to assign the measured values of the cancer markers a column value of 0 if it is within the reference range, a column value of + 1 if higher than the reference range, or a column value of - 1 if it is lower than the reference range according to the above three groups of discrimination information. For example, in a panel in the lower part of FIG. 5, the reference range is indicated by "white", a value higher than the reference range is indicated by "black", and a value lower than the reference range is indicated by "gray", and respective columns are assigned a column value of 0, + 1, or - 1.

According to the cancer test method of the present invention, a marker panel of cancer markers of a subject may be compared with the marker panel including the discrimination information in the database, and cancer can be evaluated on the basis of its homology or difference with the discrimination information of the cancer patients or the healthy subjects. In a case of a subject who cannot be determined to be a cancer patient or a healthy subject by this method, it may be preferable to make a further analysis as follows.

In one embodiment, a correlation with the discrimination information may be made on the basis of a value of an evaluation function shown in FIG. 5 and the following Formula I:

$$\text{Evaluation function} = \sum_{n=1}^{m} \pm g_n \times \sqrt{(D_n{}^2)} \qquad (I)$$

(wherein,

$g_n$ denotes a relative value of the importance calculated by machine learning, and the sign of $g_n$ is + if a measured value of a cancer marker of a subject has the same column value as that of the cancer patients and - if it has a different column value; and
$D_n$ denotes a column value of + 1, 0, or - 1).

A value obtained by the evaluation function is an estimate of the amount of variation of a cancer marker. On the basis of respective values of the evaluation function for the cancer patients and the healthy subjects in the database, an evaluation function value that can give an evaluation of being at risk for cancer and an evaluation function value that can give an evaluation of being healthy can be calculated. For example, when the evaluation function value that can give an evaluation of being at risk for cancer is denoted by $\alpha$, and the evaluation function value that can give an evaluation of being healthy is denoted by $\beta$, a subject may be evaluated to be at risk for "cancer" if an evaluation function value of the subject is above $\alpha$, and may be evaluated to be "healthy" if the evaluation function value is less than $\beta$.

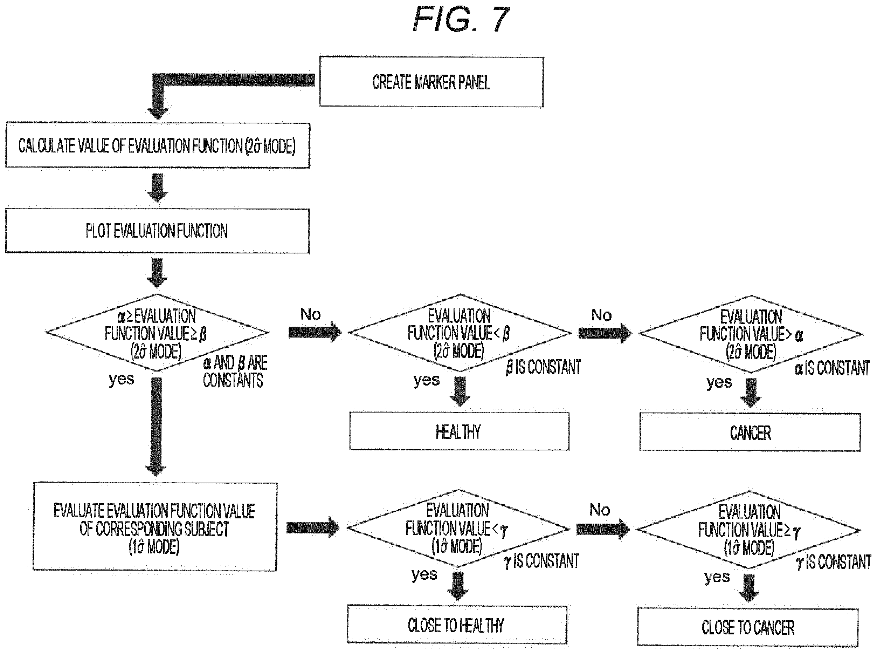

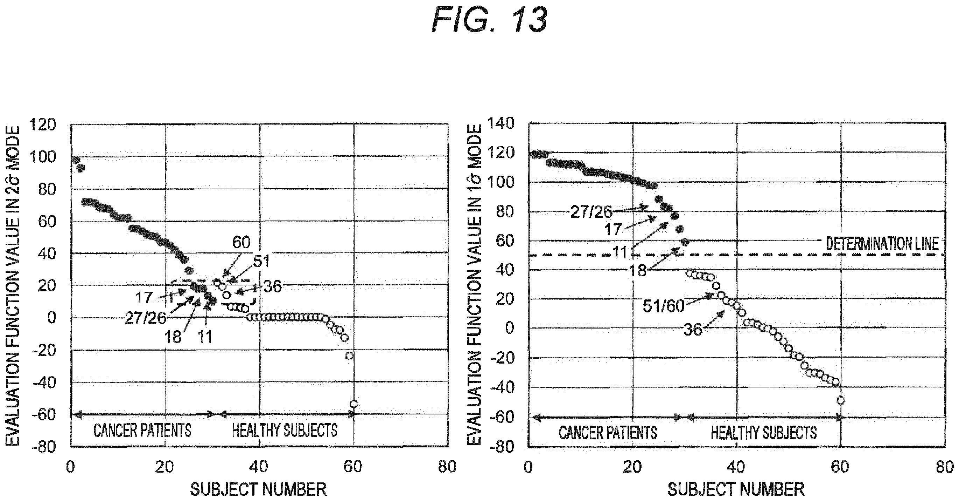

FIG. 7 shows an example of a specific flow in a case where an evaluation of cancer is made on the basis of the evaluation function. FIG. 7 is determination algorithm 1 in which analysis of a correlation with discrimination information is made by calculating an evaluation function on the basis of discrimination information with a mean value of the healthy subjects $\pm$ X $\times$ standard deviation (wherein X is 2) as a reference range, and then calculating an evaluation function on the basis of discrimination information with the mean value of the healthy subjects $\pm$ the standard deviation as a reference range. Specifically, first, an evaluation function may be calculated in 2σ mode. As described above, for example, when an evaluation function value that can give an evaluation of being at risk for cancer is denoted by $\alpha$, and an evaluation

function value that can give an evaluation of being healthy is denoted by β, a subject may be evaluated to be at risk for "cancer" if an evaluation function value of the subject is above α, and may be evaluated to be "healthy" if the evaluation function value is less than β. Here, in a case where the evaluation function value is equal to or more than β and equal to or less than α, an evaluation function may further be calculated in 1σ mode. An evaluation function plot may be created, and a reevaluation of each subject may be made using a determination line that discriminates between cancer patients and healthy subjects (for example, the determination line can be drawn as shown in FIG. 13). A subject may be evaluated to be "close to cancer" if an evaluation function value is equal to or more than γ that is a value of this determination line, and may be evaluated to be "close to healthy" if the evaluation function value is less than γ. By means of this algorithm, four evaluation results, "cancer", "healthy", "close to cancer", and "close to healthy", may be obtained.

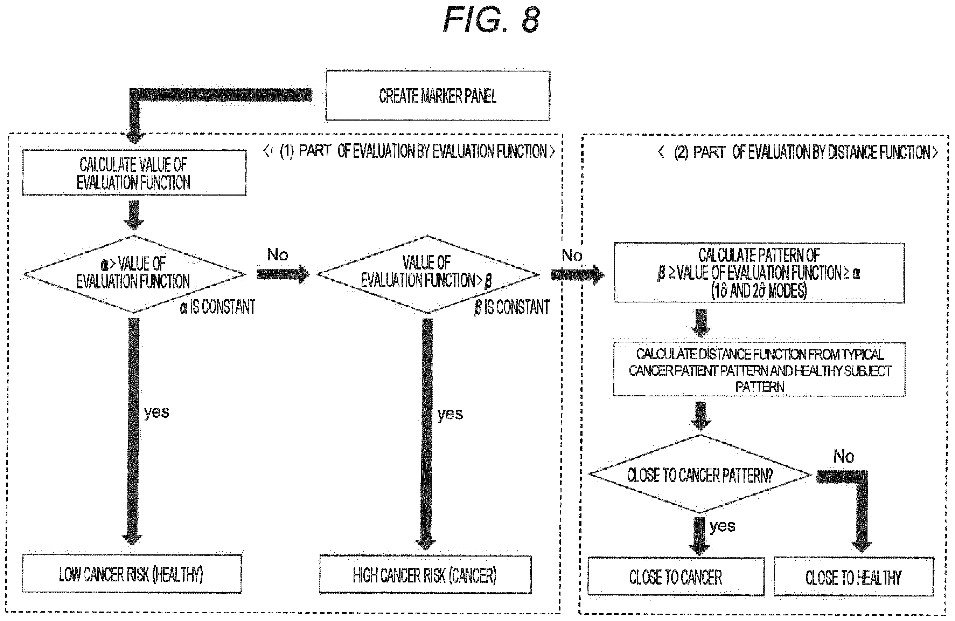

FIG. 8 shows an example of another specific flow. FIG. 8 is determination algorithm 2 that further includes: with respect to measured values of one or more cancer markers of a subject, calculating differences from a pattern of the discrimination information of cancer patients and a pattern of the discrimination information of healthy subjects in the database; and analyzing which of the pattern of the cancer patients and the pattern of the healthy subjects the measured value of the subject is close to. That is, similarities to a marker pattern of the cancer patients and a marker pattern of the healthy subjects may be analyzed. Here, the pattern means a set of discrimination information of multiple cancer markers. Preferably, after an evaluation function is calculated as described above, if a subject cannot be determined to be a cancer patient or a healthy subject, a further analysis of the pattern may be made.

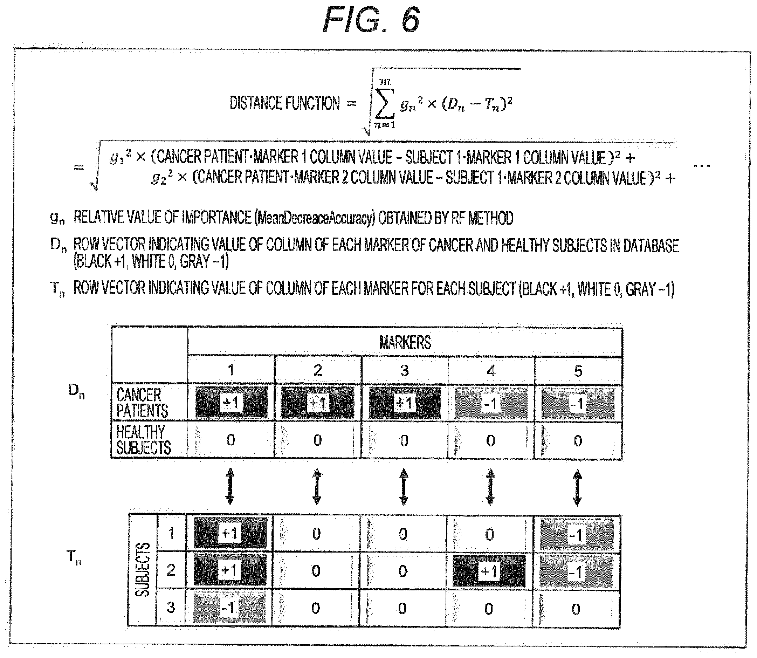

In one embodiment, a difference from the pattern can be calculated on the basis of a value of a distance function shown in FIG. 6 and the following Formula II:

$$\text{Distance function} = \sqrt{\sum_{n=1}^{m} g_n^2 \times (D_n - T_n)^2} \quad \text{(II)}$$

(wherein,

$g_n$ denotes a relative value of the importance calculated by machine learning;
$D_n$ denotes a row vector represented by a column value of + 1, 0, or - 1 with respect to each cancer marker of the cancer patients or the healthy subjects in the database; and
$T_n$ denotes a row vector represented by a column value of + 1, 0, or -1 with respect to each cancer marker of a subject).

A value obtained by the distance function is a degree of similarity of a cancer marker to the pattern. As with the determination algorithm 1, an evaluation function may be calculated on the basis of discrimination information with a mean value of the healthy subjects ± X × standard deviation (wherein X is 2) as a reference range. Specifically, first, an evaluation function may be calculated in 2σ mode. As described above, for example, when an evaluation function value that can give an evaluation of being at risk for cancer is denoted by α, and an evaluation function value that can give an evaluation of being healthy is denoted by β, a subject may be evaluated to be at risk for "cancer" if an evaluation function value of the subject is above α, and may be evaluated to be "healthy" if the evaluation function value is less than β. Here, in a case where the evaluation function value is equal to or more than β and equal to or less than α, a distance function may further be calculated. That is, a pattern of the subject may be compared with a pattern of a typical cancer patient (for example, a cancer patient with high severity) and a pattern of a typical healthy subject (for example, such as a healthy subject whose cancer markers are all within the reference range), and whether or not there is a deviation (a difference) may be calculated. The one having a smaller deviation (difference) may be an evaluation result. That is, the subject may be evaluated to be "close to cancer" if the pattern of the subject is close to the pattern of the cancer patients, and may be evaluated to be "close to healthy" if not close to the pattern of the cancer patients (the right side of FIG. 8). By means of this algorithm, four evaluation results, "cancer", "healthy", "close to cancer", and "close to healthy", may be obtained.

In the cancer test method using the metabolite list according to the present invention, four types of evaluations, "cancer", "healthy", "close to cancer", and "close to healthy", can be made, thus a cancer test becomes more precise, and it becomes possible to evaluate a subject who has been previously determined to be falsely positive or falsely negative with more precise and high accuracy.

The "evaluation" made by the cancer test method or the cancer test system according to the present invention is intended to be able to evaluate a statistically significant proportion of subjects. Therefore, the "evaluation" made by the method and the system according to the present invention also includes a case where all (i.e., 100%) of subjects cannot always have a correct result. The statistically significant proportion can be determined by means of a variety of well-known statistical evaluation tools, for example, such as determination of confidence interval, determination of p-value, Student's t-test, and Mann Whitney test. A preferred confidence interval may be at least 90%. The p-value may preferably be 0.1, 0.01, 0.05, 0.005, or 0.0001. More preferably, at least 60%, at least 80%, or at least 90% of subjects can be appropriately evaluated by the method or the system according to the present invention.

Below is described an embodiment in which urinary metabolites are used as cancer markers, and a cancer test is performed on the basis of a metabolite list.

First, urinary metabolites in a urine sample of a subject may be measured. The urinary metabolites to be measured are not particularly limited as long as they can be used as cancer markers. For example, urinary metabolites exemplified in the Examples and urinary metabolites reported in WO 2017/213246 A (colorectal cancer and breast cancer), JP 2019-168319 A, PTL 2 (pediatric cancer), etc. can be used. Multiple urinary metabolites may be used in combination, preferably, 3 or more, 5 or more, 10 or more, or 20 or more urinary metabolites may be used in combination, thereby more precise and highly accurate evaluation and monitoring of efficacy in treatment may become possible. The combination of urinary metabolites is not particularly limited, and can be appropriately selected according to the type of cancer, the sex and age of a subject, the purpose, including determination of cancer or cancer risk, follow-up (monitoring of cancer), and monitoring of treatment, etc.

The urine sample means urine collected from a subject and a sample obtained by treating the urine (for example, urine added with a preservative, such as toluene, xylene, or hydrochloric acid).

Furthermore, subjects may be humans and other mammals, for example, primates, domestic animals, animals for pets, and experimental animals, and further, may be reptiles, birds, or other things. In particular, the subject may preferably be a human. For example, the present invention may be applied to mass screening in a medical examination or a cancer test, or may be applied at the time of additional checking after such mass screening.

Measurement of a urinary metabolite means measuring the amount or concentration of the metabolite in a urine sample, preferably semiquantitatively or quantitatively, and the amount of the metabolite may be either an absolute amount or a relative amount. The measurement can be directly or indirectly made. The direct measurement may include measuring the amount or concentration of the metabolite on the basis of a signal directly correlated with the number of molecules of the urinary metabolite present in the sample. Such a signal may be based on, for example, a specific physical or chemical characteristic of the urinary metabolite. The indirect measurement may be measurement of a signal obtained from a secondary component (i.e., a component other than the urinary metabolite), for example, a ligand, a label, or an enzymatic product.

A method of measuring a urinary metabolite is not particularly limited, and a method or means publicly known in this technical field can be used. For example, measurement of a urinary metabolite can be made by a means for measuring a physical or chemical characteristic specific to the urinary metabolite, for example, a means for measuring a precise molecular weight or NMR spectrum, or the like. Means for measuring a urinary metabolite may include analyzers such as a mass spectrometer, an NMR spectrometer, a two-dimensional electrophoresis apparatus, a chromatograph, and a liquid chromatography mass spectrometer (LC/MS). These analyzers may be used independently to measure urinary tumor markers, or urinary tumor markers may be measured by a plurality of the analyzers.

Alternatively, in a case where a reagent for detecting a metabolite to be measured, for example, an immunoreaction reagent, an enzyme reaction reagent, or the like can be used, the metabolite in urine can be measured using such a reagent.

As above, a urinary metabolite contained in a urine sample collected from a subject may be measured, and a measured value of the urinary metabolite may be applied to the above cancer test method of the present invention, thereby it is possible to evaluate cancer in the subject. Furthermore, the urinary metabolite in each of urine samples collected at multiple points of time from the subject may be measured.

Then, with respect to the above measured value of the urinary metabolite of the subject, a correlation with the discrimination information in the database may be analyzed. The database here has stored therein a metabolite list on which information of multiple urinary metabolites with respect to multiple healthy subjects and cancer patients may be registered. The database may include, with respect to the measured value of each urinary metabolite, discrimination information that classifies the urinary metabolite, with

a mean value of the healthy subjects $\pm$ X $\times$ standard deviation (wherein X is an arbitrary numerical value) and/or the mean value of the healthy subjects $\pm$ the standard deviation

as a reference range, into any of three groups: within the reference range, higher than the reference range, and lower than the reference range.

In one embodiment, with respect to the measured value of the urinary metabolite of the subject, a correlation with three groups of discrimination information may be analyzed. As described above, the analysis of the correlation with the discrimination information can be made, for example, by comparison of metabolite lists by means of the determination algorithm 1 or the determination algorithm 2. On the basis of a result of this analysis, cancer in the subject may be evaluated.

According to the cancer test method of the present invention, the presence or progression of cancer can be determined at an early stage with high accuracy, and cancer can be precisely evaluated and subdivide into cancer or close to cancer, or healthy or close to healthy. For example, it may also be possible to evaluate the stage or severity of cancer, and this helps in determining an extensive examination and a treatment plan. If a simple test makes it possible to diagnose whether or not cancer is present or if a subject is at risk for cancer, it can be expected that not only treatment but also invasion risk caused by a test can be prevented. The subject can receive treatment for cancer early and a follow-up after the treatment. Furthermore, it may be possible to monitor the efficacy in the treatment of cancer, and it may be possible to consider the discontinuance, continuance, or change of the treatment according to the efficacy in the treatment. Moreover, since a urine sample is used, a minimally invasive evaluation of cancer can be made with ease and at low cost.

In another embodiment, a urine sample may be collected from a subject at multiple points of time, a urinary metabolite contained in the urine sample at each point of time of measurement may be measured, and, with respect to a measured value of the urinary metabolite at each point of time of measurement, a correlation with discrimination information in the database may be analyzed. The measurement can be made at least 2 times, 3 times, 4 times, 5 times, 10 times, 15 times, 20 times, 30 times, or more than 30 times with time, for example, at intervals of 1 day, 2 days, 5 days, 1 week, 2 weeks, 3 weeks, 1 month, 2 months, 3 months, half a year, 1 year, 2 years, 3 years, 5 years, or more than 5 years. By this analysis, time-course monitoring can be performed, and the progress of cancer, the metastasis or recurrence of cancer, the onset of cancer from no abnormalities, etc. can be evaluated.

In still another embodiment, efficacy in treatment (a therapeutic agent or method) of cancer in a subject having cancer can be monitored. Specifically, it includes:

(a) a step of measuring urinary metabolites in a urine sample from a subject having cancer before the subject undergoes a treatment with a therapeutic agent or method;
(b) a step of measuring the urinary metabolites in a urine sample from the subject having cancer after the subject undergoes the treatment with the therapeutic agent or method;
(c) a step of repeating the step (b) as necessary; and
(d) a step of monitoring efficacy of the therapeutic agent or method on cancer on the basis of analysis of measured values obtained in (a) to (c).

In the above method, a urine sample may be collected from a subject having cancer before the subject undergoes a treatment with a therapeutic agent or method, and urinary metabolites in the urine sample may be measured. After the treatment with the therapeutic agent or method is performed on the subject having cancer, a urine sample may be collected at an appropriate time, and the urinary metabolites in the urine sample may be measured. For example, a urine sample may be collected immediately, 30 minutes, 1 hour, 3 hours, 5 hours, 10 hours, 15 hours, 20 hours, 24 hours (1 day), 2 to 10 days, 10 to 20 days, 20 to 30 days, and 1 month to 6 months after the treatment. Measurement of urinary metabolites in the urine sample can be made in a similar way to the above. By measuring urinary metabolites before and after the treatment, it becomes possible to monitor the efficacy in the treatment with the therapeutic agent or method. This helps to consider the discontinuance, continuance, or change of the treatment on the basis of a result of the monitoring.

Furthermore, the cancer test method may be performed in combination with other conventional publicly known methods for the diagnosis of cancer. Such publicly known methods for the diagnosis of cancer include an imaging test (for example, such as ultrasonography, computer tomography (CT), X-ray radiography, and positron CT (PET)), endoscopy, a pathological examination with a biopsy, measurement of cancer markers in blood, etc.

On the basis of a result of the above evaluation, a doctor can make a diagnosis of the subject's cancer and perform an appropriate treatment. That is, the present invention also relates to a method of examining cancer in a subject and treating it. For example, in a case where cancer in a subject has been determined in accordance with the method according to the present invention, and the subject has been evaluated to be highly likely to have cancer, a treatment for treating cancer or preventing the progression of cancer in the subject may be performed. Furthermore, in a case where it has been evaluated that the stage of cancer in the subject has progressed or that cancer is highly likely to have a poor prognosis, the treatment may be continued, or a change in the method may be considered if necessary. Moreover, in a case where it has been evaluated that there is a high possibility that cancer is present in the subject, the presence of cancer may be confirmed by performing another method for the diagnosis of cancer such as the above methods. Furthermore, on the basis of results of the evaluations before and after the treatment, the efficacy in the treatment may be monitored, and the discontinuance, continuance, or change of the treatment may be determined. Moreover, in a case where it has been determined that there are no abnormalities, measurement of urinary metabolites can be made with time to follow up.

As a method for the treatment of cancer, surgery, radiotherapy, chemotherapy, immunotherapy, proton beam therapy, heavy ion radiotherapy, etc. can be performed either alone or in combination appropriately. The treatment of cancer can be appropriately selected by those skilled in the art in consideration of the type of cancer, the stage, the severity, the malignancy, the sex, the age and condition, the responsiveness to treatment, the genetic polymorphism (SNP) carried, etc.

The cancer test method of the present invention can be easily and simply performed by using a system. A cancer test system according to the present invention includes the following means:

a storage unit including a database that has stored a marker panel on which information of multiple cancer markers with respect to multiple healthy subjects and cancer patients is registered, the database including discrimination information that classifies a measured value of each cancer marker, with a mean value of the healthy subjects $\pm$ X $\times$ standard deviation (wherein X is an arbitrary numerical value) and/or the mean value of the healthy subjects $\pm$ the standard deviation as a reference range, into three groups: within the reference range, higher than the reference range, and lower than the reference range;

an input unit that is configured to receive inputs of measured values of one or more cancer markers of a subject;

an analysis unit that is configured to analyze, with respect to the measured values of the one or more cancer markers of the subject from the input unit, a correlation with the discrimination information in the database; and

an evaluation unit that is configured to evaluate cancer in the subject on the basis of an analysis result obtained by the analysis unit.

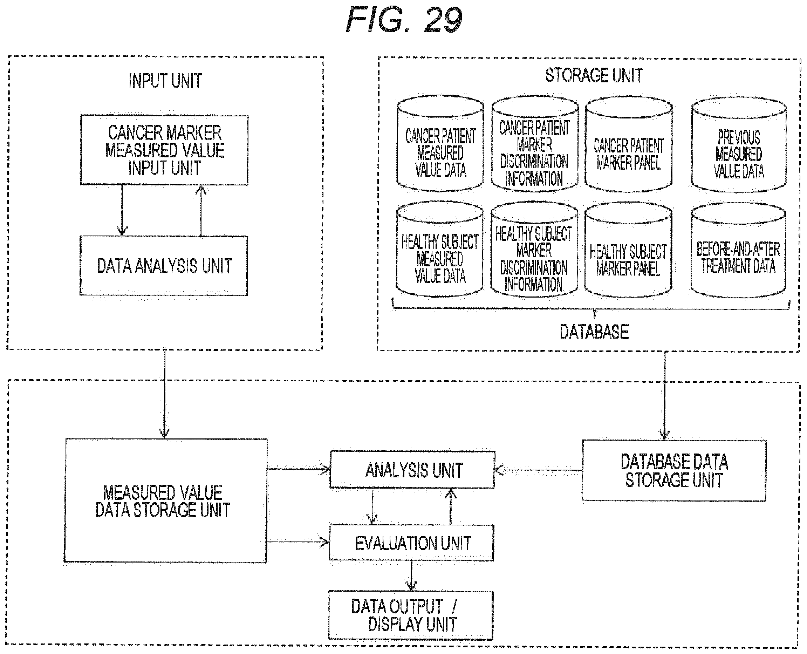

The system of the present invention may preferably be a system in which the storage unit, the input unit, the analysis unit, and the evaluation unit described above are operably connected to one another so that the cancer test method of the present invention can be implemented. FIG. 29 shows an embodiment of the system of the present invention.

Here, the input unit may be configured to receive an input of a measured value of a cancer marker; a user may input the measured value through any input device, or may import the measured value obtained by another device (for example, an apparatus for measuring a cancer marker, such as a mass spectrometer, an NMR spectrometer, or a liquid chromatography mass spectrometer (LC/MS) apparatus as an example), or may retrieve the measured value input to another system. The input unit may include a data analysis unit that is configured to analyze a measured value of a cancer marker of which the input has been received. For example, it may be configured to analyze whether data of the type of cancer marker, a measured value, subject information, etc. is suitable for subsequent analysis, and/or to perform data pretreatment.

The database of the storage unit has stored a marker panel on which information of multiple cancer markers with respect to multiple healthy subjects and cancer patients may be registered. The database may include, as information of the cancer markers, with respect to each of the cancer patients and the healthy subjects, at least the types of the cancer markers, the measured values of the cancer markers, and the discrimination information. Preferably, a cancer marker panel of the cancer patients and the healthy subjects may be stored in the database. Furthermore, the database may store data of multiple cancer markers with respect to cancer patients in a specific stage or with a specific degree of severity, or may store data of previous measured values or data of cancer patients before and after treatment. Furthermore, the database may include different databases according to the types of cancers.

The analysis unit may include a data analysis unit including software for processing a measured value obtained from the input unit and a calculator. The data analysis unit may be configured to create a marker panel of a subject on the basis of the measured value obtained from the input unit. The data analysis unit can include, for example, a signal display portion, a unit for analyzing a measured value, a computer unit, etc.

Furthermore, the analysis unit may be configured to read out information (for example, the discrimination information) of the cancer markers of the cancer patients and the healthy subjects from a storage device (a database) or something, and to analyze a correlation with measured values of cancer markers of a subject of which the inputs have been received by the input unit. At this time, the analysis unit may be configured to selectively read out an appropriate database according to the type of the cancer markers. Alternatively, in a case of temporal monitoring in the same subject, the analysis unit may be configured to read out the previous measured values from the storage device (the database) or something, and to compare the previous measured values with the measured values of the cancer markers of which the inputs have been received by the input unit.

Furthermore, the evaluation unit may be configured to evaluate cancer in the subject on the basis of a result of the analysis of the correlation made by the analysis unit. Here, the evaluation unit may be configured to acquire information indicating the presence or absence of cancer in the subject, the stage of cancer, no abnormalities, etc. For example, information that the subject is "cancer", "healthy", "close to cancer", or "close to healthy" may be acquired. A preferred system may be a system that a user can use even without knowledge of a specialized clinician, and, for example, when data of measured values has been input to an input unit, the data is automatically analyzed, and a result of evaluation of cancer in a subject is displayed. At that time, together with the result of evaluation of cancer, a marker panel (a metabolite list) of the analyzed cancer markers may be displayed together.

The cancer test system of the present invention may further have a data storage unit, a data output and display unit, etc.

As an example of application of the present invention, a cancer test in a test center is described. In the test center, information of a cancer test is provided in response to a request or the like from a test subject. The test subject may make a choice about the number of markers for a test when applying for a primary test. This can also be used as an overall cancer test (various cancers are analyzed at a time) in combination with other markers.

Then, the test center delivers the test subject a test kit necessary for collection of urine. The test kit is sent by mail or the like as necessary. After the test subject receives the test kit, the test subject delivers or sends a specimen to the test center, or does it in some other way. In the test center, the specimen is cryonically preserved at about -80°C as necessary for a subsequent test. In the test center, a primary test is performed according to the cancer test method described above, and a test result is sent to the test subject.

The test subject receives the result of the primary test, and may apply for a secondary test according to the content, or may have a more detailed diagnosis. This makes it possible to confirm the suspicion of cancer in the primary test and further identify the stage of cancer.

The present invention is specifically described below with examples; however, these examples are merely provided for description of the present invention, and do not limit or restrict the scope of the invention disclosed in the present application.

[Examples]

[Example 1] Example of Evaluation of Colorectal Cancer (Determination Algorithm 1)

As an example showing the efficacy of the present method, evaluations of colorectal cancer in subjects of Mongoloid descent (30 colorectal cancer patients and 30 healthy subjects) were made. Urine samples were collected from the subjects, and urinary metabolites were detected by means of a liquid chromatography mass spectrometry (LC/MS).

(1) Creation of Metabolite List

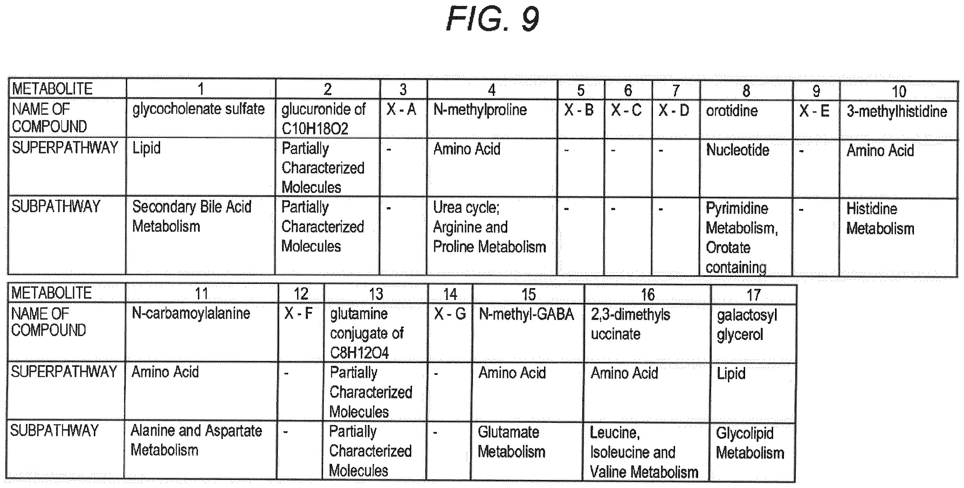

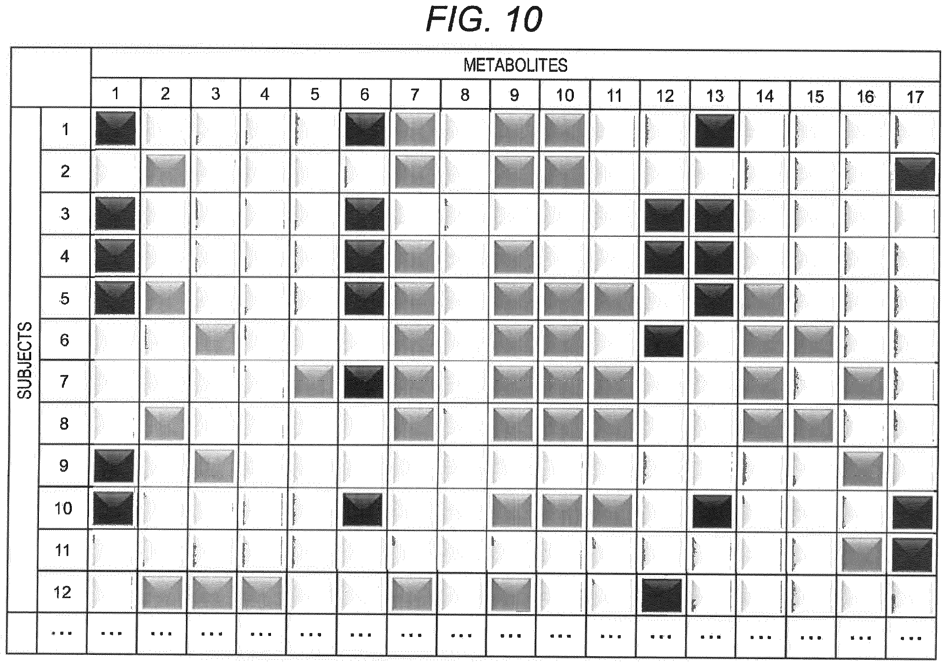

With 60 subjects (30 colorectal cancer patients and 30 healthy subjects) on the vertical axis and 17 types of urinary tumor markers (metabolites) on the horizontal axis, a metabolite list was created. FIG. 9 shows the types of 17 urinary tumor markers (metabolites) used at that time. It is noted that X in an item of NAME OF COMPOUND denotes a compound under structural analysis. In FIG. 9, a superpathway means a biological pathway of the same series of whole metabolites, and a subpathway means their individual biological pathways. For example, tyrosine metabolism is a metabolic pathway centered on tyrosine and is a subpathway; a plurality of metabolic pathways related to amino acids is collectively referred to as an amino acid metabolic pathway, and this is a superpathway. FIG. 10 shows a part (evaluation based on an evaluation function in $2\sigma$ mode) as an example of the obtained metabolite list. Specifically, with a mean value of the healthy subjects $\pm$ 2 $\times$ standard deviation as a reference range, a column of a marker of which the measured value of a metabolite was higher than the reference range was displayed in black; a column of a marker within the reference range was displayed in white; and a column of a marker lower than the reference range was displayed in gray.

(2) Creation of Evaluation Function Plot

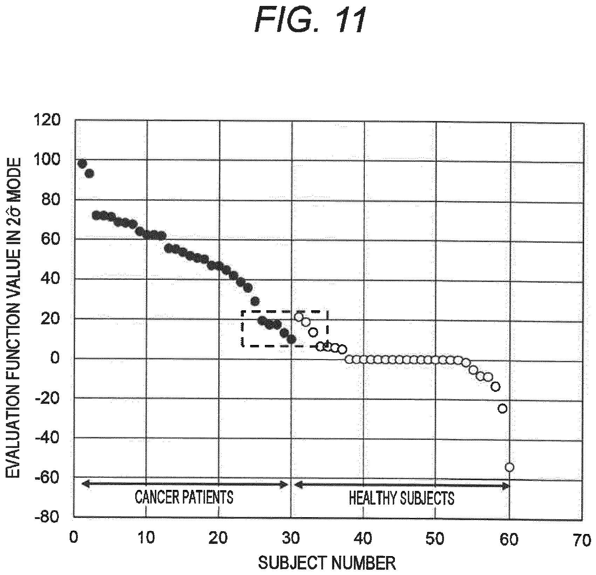

As shown in FIG. 11, an evaluation function plot was created in $2\sigma$ mode for the purpose of discriminating between the colorectal cancer patients and the healthy subjects by the value of an evaluation function. The evaluation function was calculated by assigning a column value of + 1 to a column (black) of a marker of which the measured value of a metabolite was higher than the reference range, assigning 0 to a column (white) of a marker within the reference range, and assigning -1 to a column (gray) of a marker lower than the reference range. As indicated by a dotted frame, it can be seen that there is an area where respective evaluation function values of cancer patients and healthy subjects overlap in a range of evaluation function values of 20 to 10.

(3) Analysis by Determination Algorithm 1

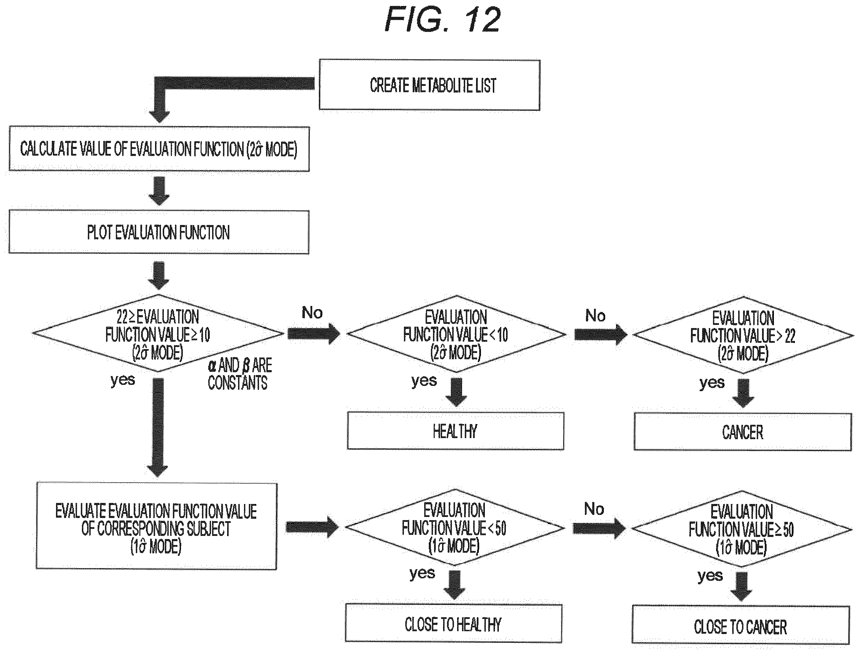

On the basis of the plot in $2\sigma$ mode, an evaluation function plot in $1\sigma$ mode was created (the right side of FIG. 13). That is, with the mean value of the healthy subjects $\pm$ the standard deviation as a reference range, respective measured values of the markers were classified into three groups of discrimination information, and were assigned column values, and then the evaluation function was calculated. FIG. 12 shows a flow of analysis by the determination algorithm 1 based on the results in $2\sigma$ mode and $1\sigma$ mode. FIG. 13 shows the evaluation function plots in $2\sigma$ mode and $1\sigma$ mode.

In $2\sigma$ mode, when an area where the evaluation function was greater than 22 was determined to be "cancer", and an area where the evaluation function was smaller than 10 was determined to be "healthy", the key point was what to make of subjects 11, 17, 18, 26, 27, 36, 51, and 60 of which the evaluation function was between 22 and 10 (the left side of FIG. 13). Accordingly, looking at the evaluation function plot in 1σ mode, it can be seen that the change in metabolites of each subject was more emphasized, and subjects 11, 17, 18, 26, 27, 36, 51, and 60 were more widely distributed (the right side of FIG. 13). Thus, when the determination line was set to an evaluation function value of 50, subjects 11, 17, 18, and 26 were able to be determined to be "close to cancer", and subjects 36, 51, and 60 were able to be determined to be "close to healthy".

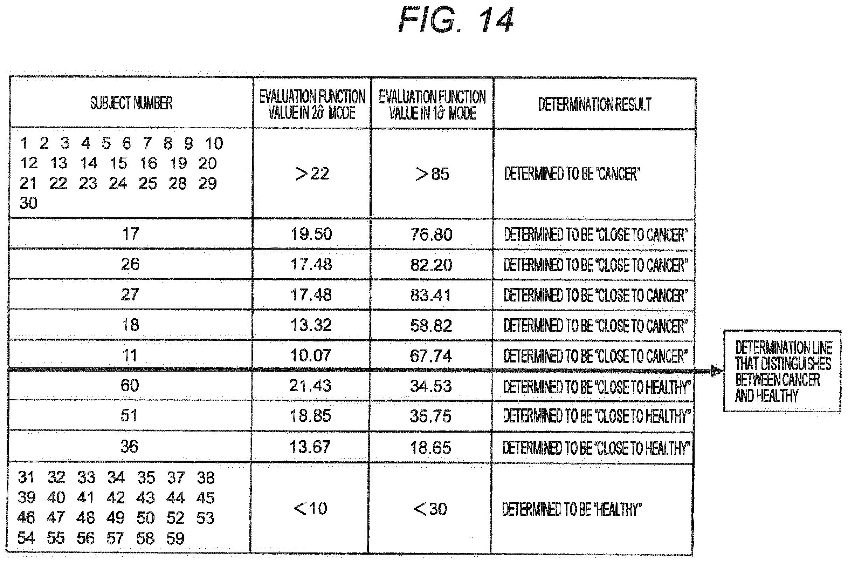

The above determination results are summarized in FIG. 14. The results shown in FIG. 14 are:

$$\text{Sensitivity} = (30/30) \times 100 = 100\%,$$

$$\text{Specificity} = (30/30) \times 100 = 100\%.$$

Therefore, by the present method, cancer was able to be evaluated with high accuracy in both sensitivity and specificity.

[Example 2] Example of Evaluation of Colorectal Cancer (Determination Algorithm 2)

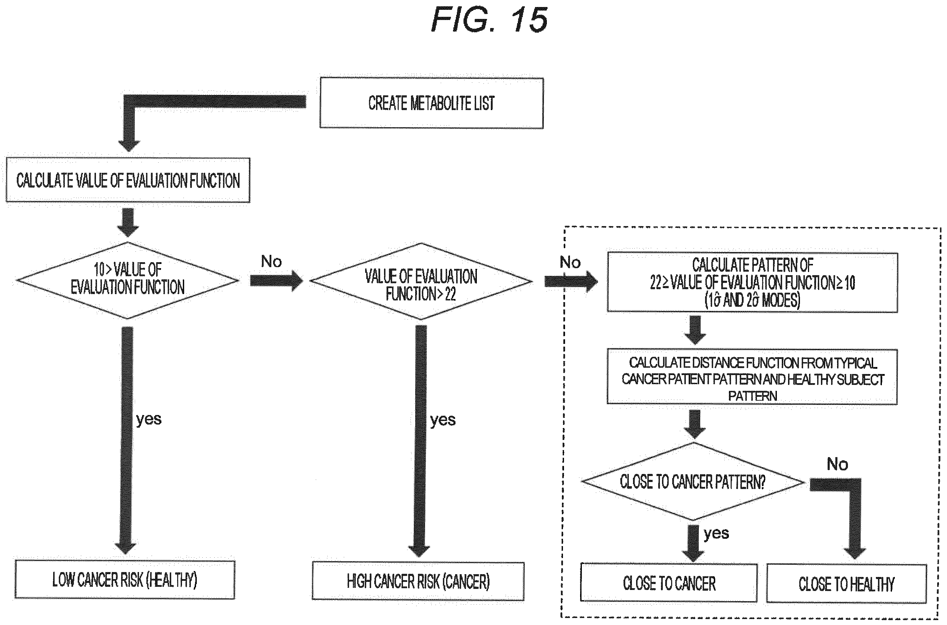

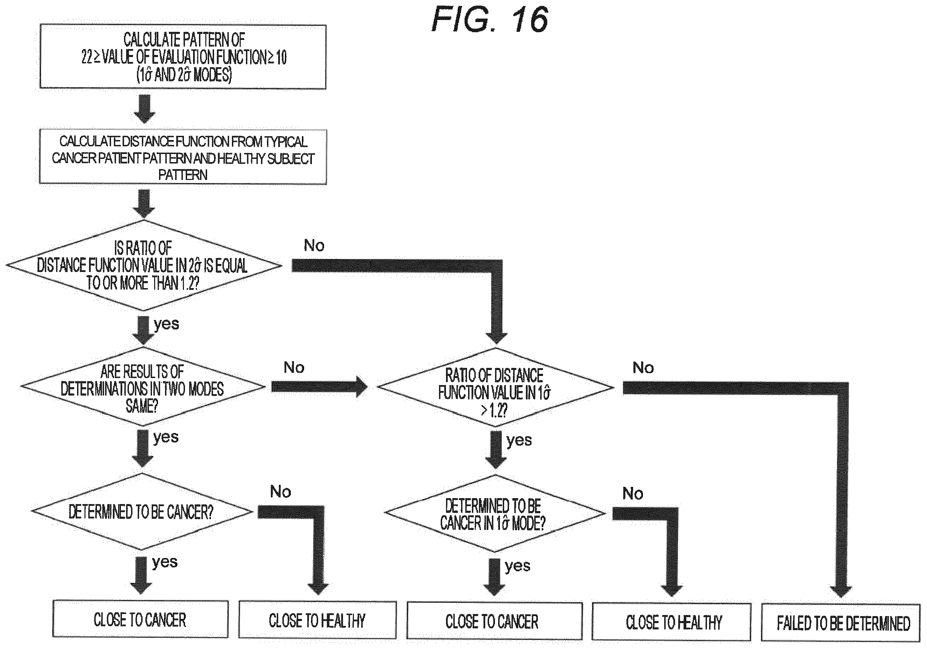

There is described an example where colorectal cancer was evaluated using a distance function in addition to the evaluation function. In a case where whether a subject is a cancer patient or a healthy subject is determined by the distance function based on the determination algorithm 2, flows shown in FIGs. 15 and 16 can be used.

First, as with the determination algorithm 1, in the evaluation function plot calculated in 2σ, an area showing a high value considered to correspond to a cancer patient (the area where the evaluation function value was greater than 22) was determined to be "cancer", and an area showing a low value considered to correspond to a healthy subject (the area where the evaluation function value was smaller than 10) was determined to be "healthy".

Next, as for an area (22 ≥ the evaluation function value ≥ 10) where it was considered that cancer patients and healthy subjects were mixed, a distance function shown in FIG. 6 was calculated (a part within the dotted frame of FIG. 15 and FIG. 16). This means that the magnitude of a deviation (a distance) from each of the metabolite list patterns of the typical cancer patient (for example, a cancer patient with high severity) and the typical healthy subject (for example, such as a healthy subject whose metabolites are all in the reference range) in the database was calculated with respect to each subject. Which of the patterns of the cancer patients and the healthy subjects that value was close to was determined. When the distance function is calculated, a distance function in 2σ mode may be calculated, and further a distance function in 1σ mode may be calculated. In FIG. 16, the "ratio of the distance function value" means a value obtained by dividing, of the distance from the typical cancer patient and the distance from the typical healthy subject, a larger value by a smaller value; the "typical cancer patient pattern" means a late-stage cancer patient; and the "typical healthy subject pattern" means a healthy subject whose urinary tumor markers (metabolites) are all within the reference range.

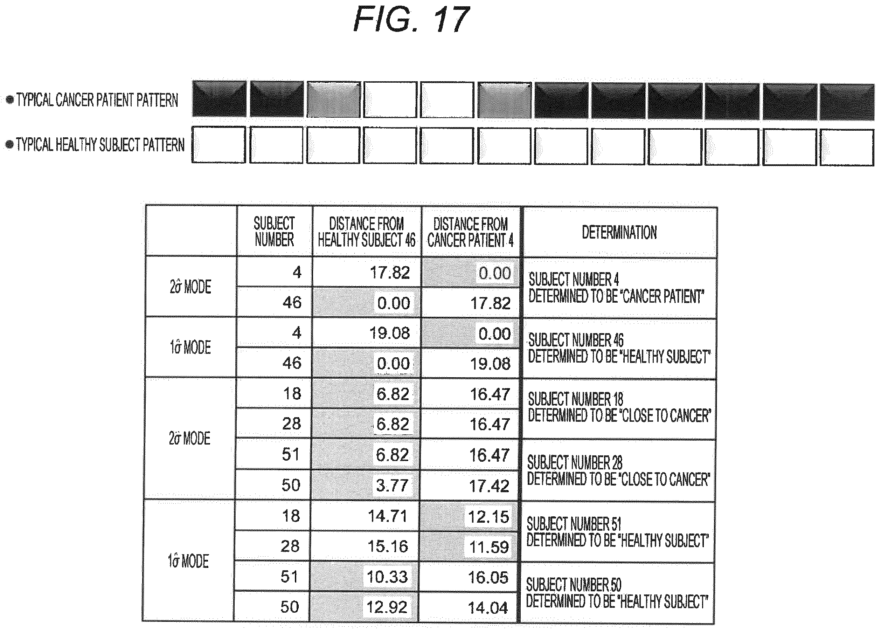

FIG. 17 shows a result of actual analysis according to the determination algorithm 2. FIG. 17 is a result of calculating a distance function (in this case, the magnitude of a deviation is indicated) from a typical colorectal cancer patient with high severity (a case where almost all metabolites of the urinary tumor markers are outside the reference range) and a typical healthy subject (a case where all metabolites of the urinary tumor markers are within the reference range). In the area (22 ≥ the evaluation function value ≥ 10) where it was considered that cancer patients and healthy subjects were mixed, subjects 18, 28, 51, and 50 were present. From the result of the distance function, while subjects 18 and 28 were determined to be "close to cancer", subjects 51 and 50 were determined to be "close to healthy".

Also in this algorithm, the results were:

$$\text{Sensitivity} = (30/30) \times 100 = 100\%,$$

$$\text{Specificity} = (30/30) \times 100 = 100\%.$$

Therefore, by the present method, cancer was able to be evaluated with high accuracy in both sensitivity and specificity.

[Example 3] Example of Evaluation of Colorectal Cancer (Evaluation of Cancer Severity by Metabolite List)

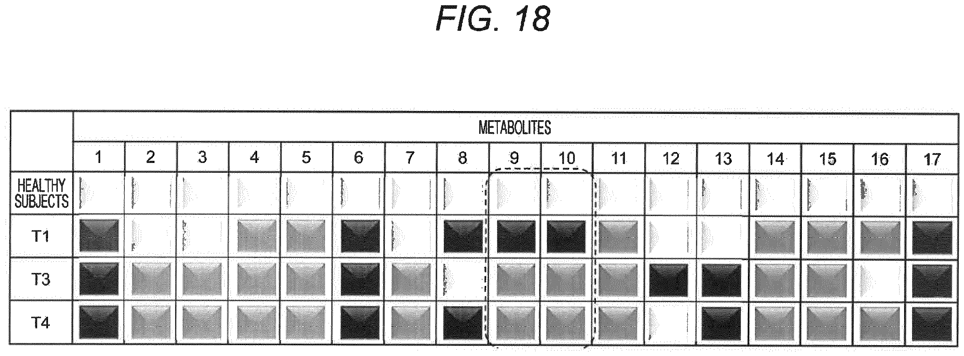

There may be a case where information about the severity of cancer is obtained by inspecting a metabolite list. FIG. 18 shows a pattern change of a metabolite list of a cancer patient with a specific depth of invasion (T) according to the TNM classification in 1σ mode with respect to urinary tumor markers (metabolites) for colorectal cancer as with Example 1. It can be seen that there is a change in the cancer pattern by the severity (the depth of invasion). For example, when metabolites 9 and 10 were observed, naturally, for healthy subjects, all metabolite lists were "white"; however, they were "black" in $T_1$ (in a case where the depth of invasion according to the TNM classification of cancer remains on the mucosa or the submucosa) and "gray" in $T_2$ (the depth of invasion is within the muscularis propria), $T_3$ (the depth of invasion is outside the muscularis propria), and $T_4$ (the depth of invasion is the serosa or outside the serosa). Therefore, it can be seen that the severity of cancer, i.e., the depth of invasion according to the TNM classification can be grasped to some extent from a pattern of a metabolite list.

[Example 4] Example of Evaluation of Colorectal Cancer (Evaluation of Cancer Severity Based on Evaluation Function)

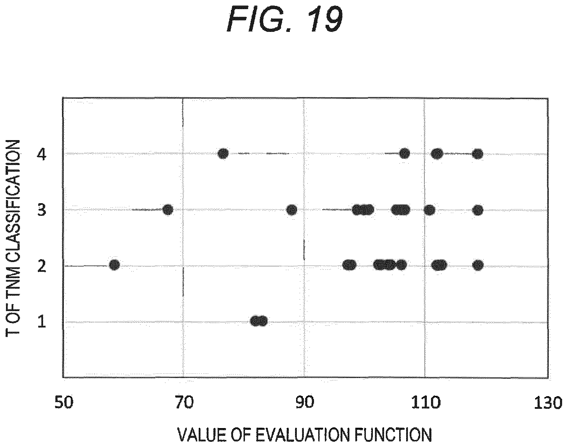

The characteristic feature of a cancer test based on an evaluation function is that it can be detected regardless of $T_1$, $T_2$, $T_3$, and $T_4$ indicating the depth of invasion according to the TNM classification. FIG. 19 shows a relationship between the value of the evaluation function in $1\sigma$ mode and $T_1$ to $T_4$ of the TNM classification. It shows a tendency that the higher the degree of severity, the larger the value of the evaluation function, and it was found that information on the severity can be obtained by the evaluation function.

[Example 5] Example of Evaluation of Breast Cancer (Determination Algorithms 1 and 2)

As an example showing the efficacy of the present method, evaluations of breast cancer (in 30 breast cancer patients (25 pretreatment patients and 5 posttreatment patients) and 210 healthy subjects) were made. Urine samples were collected from the subjects, and urinary metabolites were detected by means of a liquid chromatography mass spectrometry (LC/MS). In the determination algorithm 1 and the determination algorithm 2, the efficacy similar to that of the case of colorectal cancer was obtained.

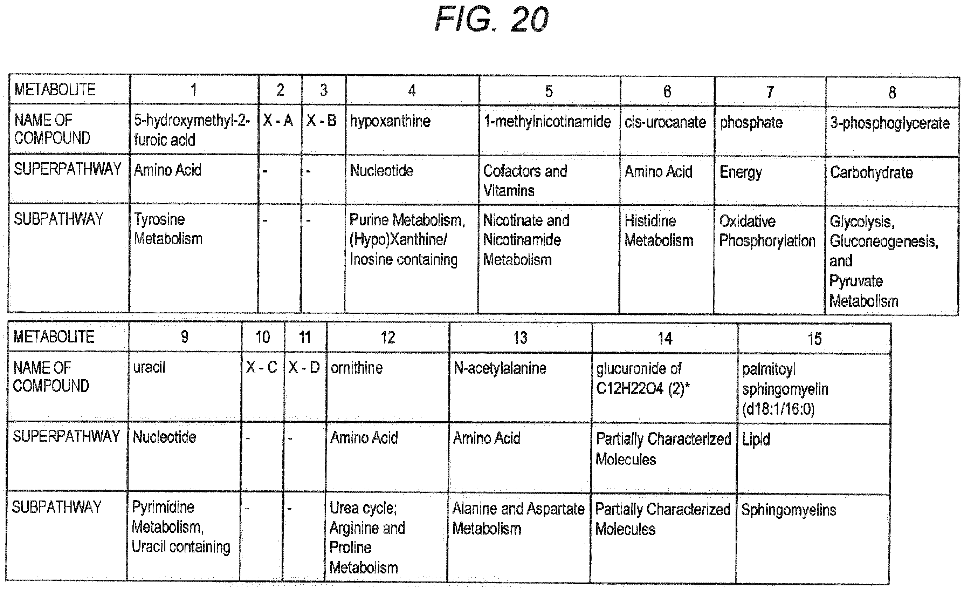

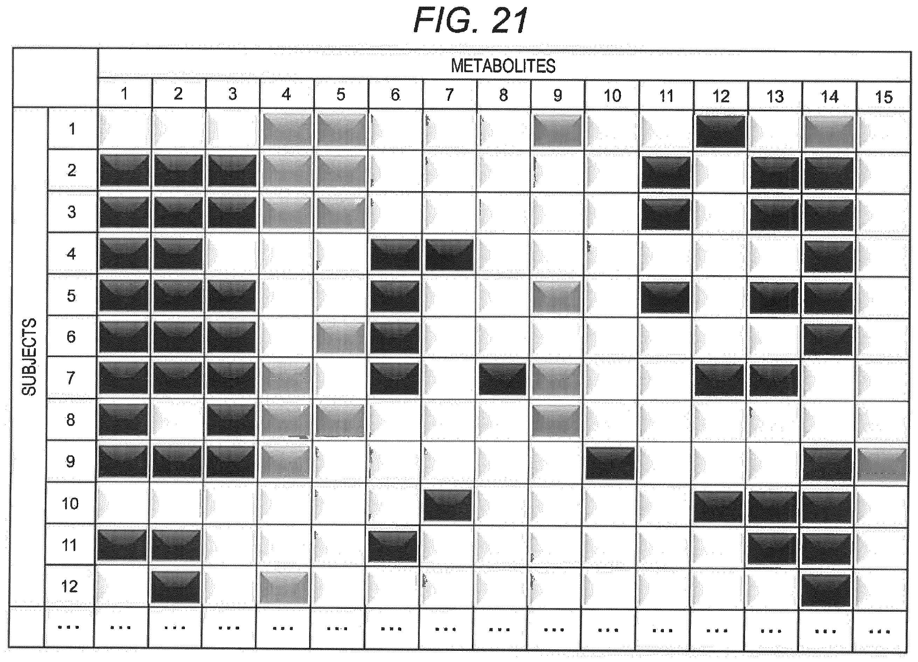

FIG. 20 shows the types of 15 urinary tumor markers (metabolites) used. It is noted that X in an item of NAME OF COMPOUND denotes a compound under structural analysis. The superpathway and the subpathway are similar to those in Example 1. FIG. 21 shows a part (evaluation based on the evaluation function in $2\sigma$ mode) as an example of the created metabolite list. Specifically, with a mean value of the healthy subjects $\pm$ 2 $\times$ standard deviation as a reference range, a column of a marker of which the measured value of a metabolite was higher than the reference range was displayed in black; a column of a marker within the reference range was displayed in white; and a column of a marker lower than the reference range was displayed in gray.

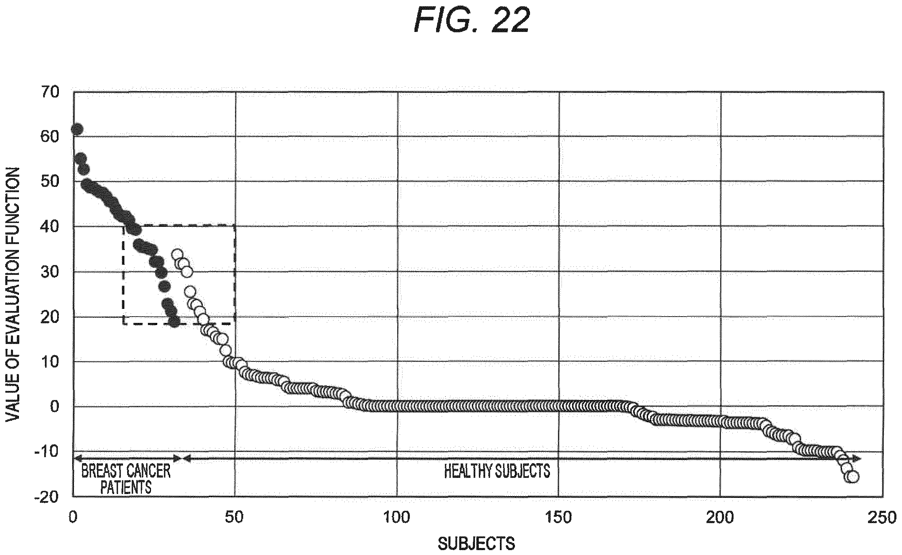

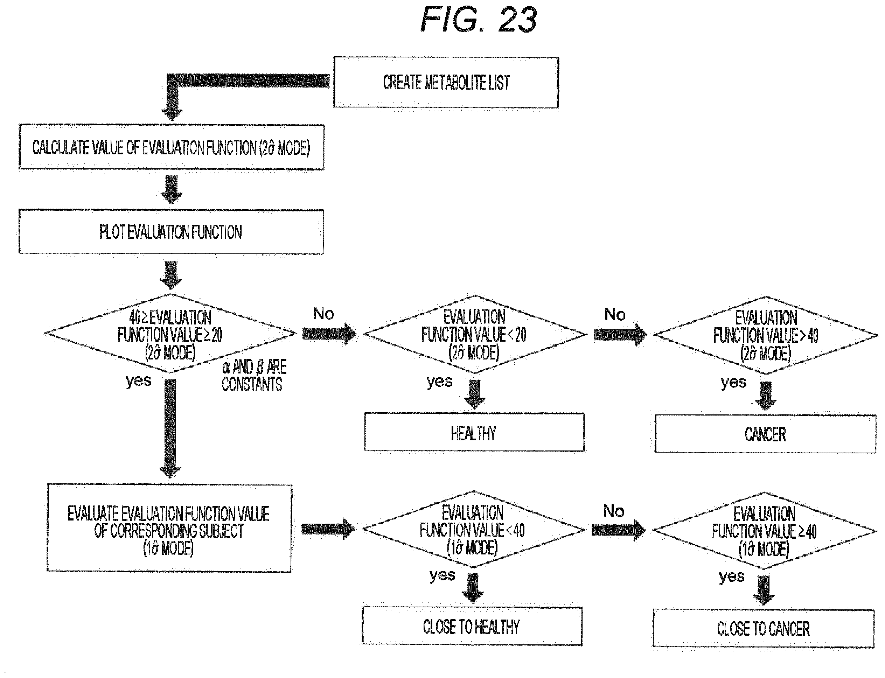

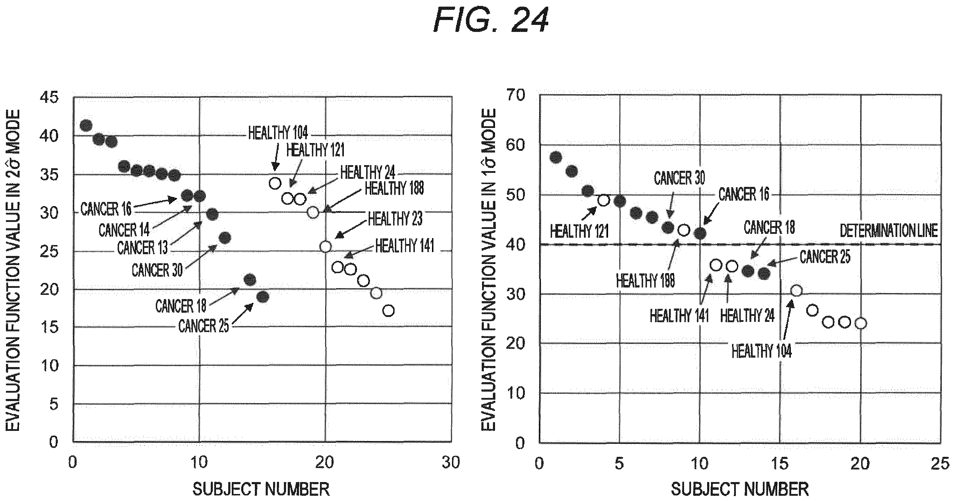

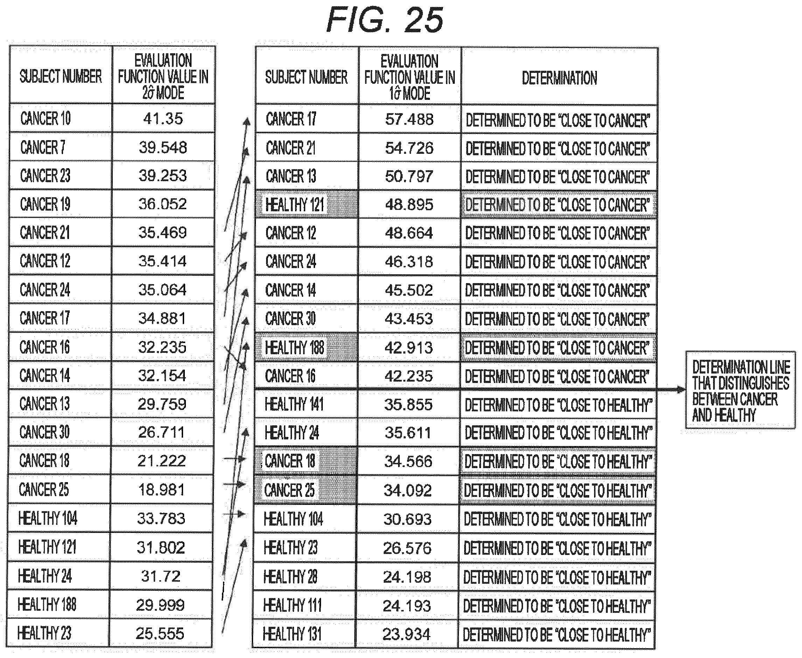

FIG. 22 shows an evaluation function plot created in $2\sigma$ mode for the purpose of discriminating between the breast cancer patients and the healthy subjects by the value of the evaluation function. As indicated by a dotted frame, it can be seen that there is an area where respective evaluation function values of cancer patients and healthy subjects overlap in a range of evaluation function values of 40 to 20. On the basis of the plot in $2\sigma$ mode, an evaluation function plot in $1\sigma$ mode was created (the right side of FIG. 24). FIG. 23 shows a flow of analysis by the determination algorithm 1 based on the results in $2\sigma$ mode and $1\sigma$ mode. FIG. 24 shows the evaluation function plots in $2\sigma$ mode and $1\sigma$ mode. In FIGs. 24 and 25, "cancer" denotes a cancer patient number, and "healthy" denotes a healthy subject number.

The determination results by the determination algorithm 1 are summarized in FIG. 25. The results shown in FIG. 25 are:

$$\text{Sensitivity} = (28/30) \times 100 = 93.3\%,$$

$$\text{Specificity} = (208/210) \times 100 = 99.0\%.$$

However, it was determined that as for subjects 18 and 25 who are cancer patients determined to be close to healthy in the sensitivity, remeasurement is required for subject 18 because there was missing data, and subject 25 is under postoperative chemotherapy, thus a future evaluation is required. Therefore, by the present method, cancer was able to be evaluated with high accuracy in both sensitivity and specificity.

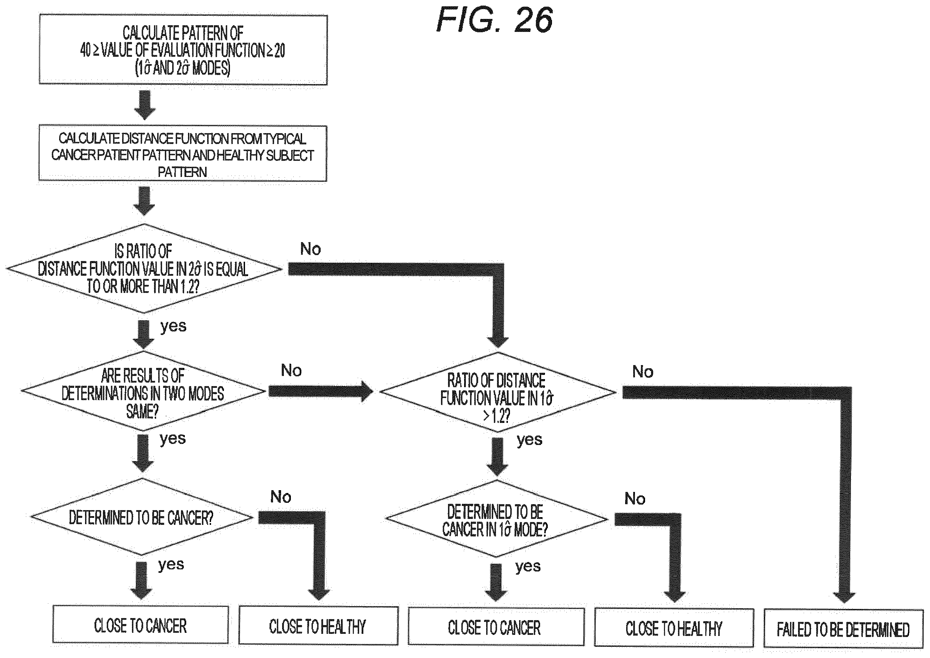

In an example where breast cancer is evaluated using a distance function in addition to the evaluation function, a flow shown in FIG. 26 can be used as the determination algorithm 2.

First, as with the determination algorithm 1, in the evaluation function plot calculated in $2\sigma$, an area showing a high value considered to correspond to a cancer patient (an area where the evaluation function value was greater than 40) was determined to be "cancer", and an area showing a low value considered to correspond to a healthy subject (an area where the evaluation function value was smaller than 20) was determined to be "healthy". Next, as for an area (40 $\geq$ the evaluation function value $\geq$ 20) where it was considered that cancer patients and healthy subjects were mixed, the distance function shown in FIG. 6 was calculated. In FIG. 26, the "ratio of the distance function value" means a value obtained by dividing, of the distance from a typical cancer patient and the distance from a typical healthy subject, a larger value by a smaller value.

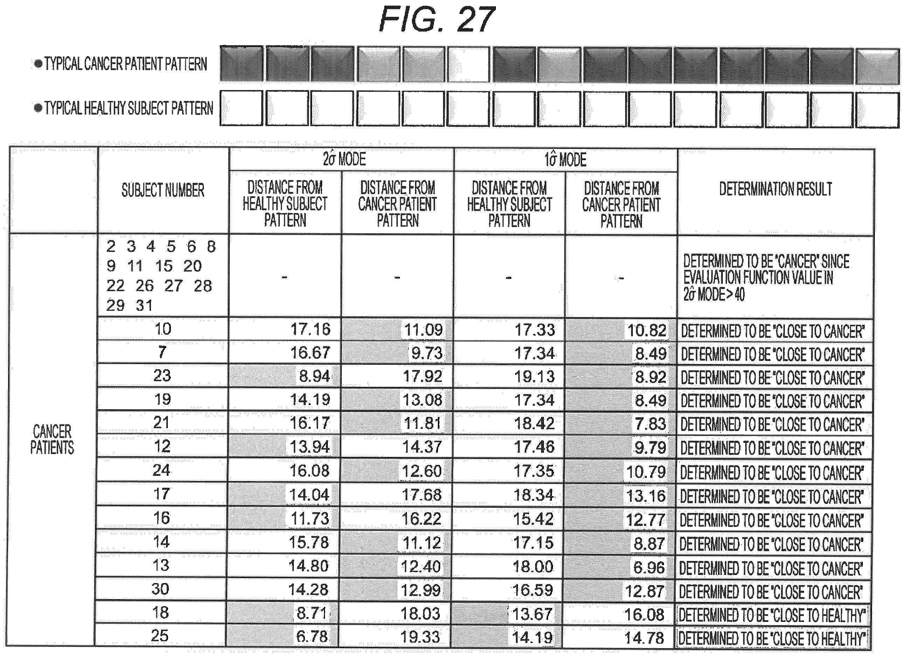

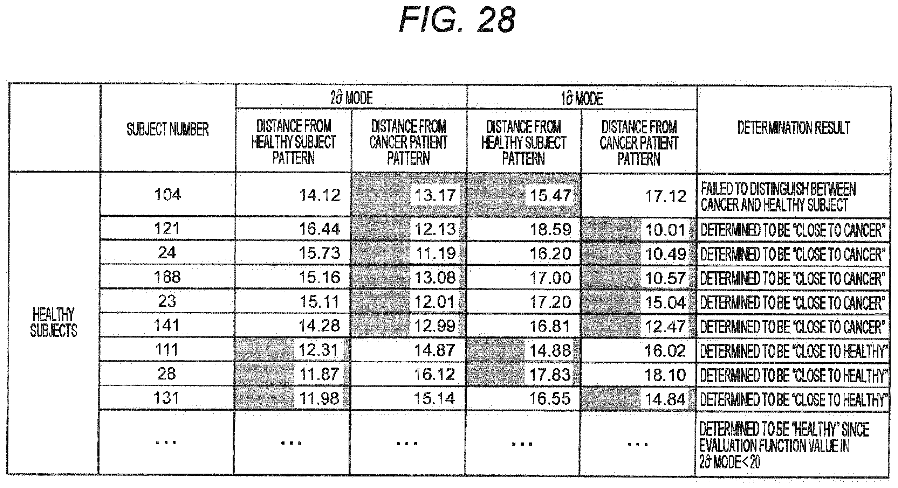

Results of actual analysis according to the determination algorithm 2 are shown in FIG. 27 (cancer patients) and FIG. 28 (healthy subjects). In a table of FIG. 27, subjects 2, 10, 17, 25, and 30 are posttreatment patients. In the area (40 ≥ the evaluation function value ≥ 20) where it was considered that cancer patients and healthy subjects were mixed, cancer patients 10, 7, 23, 19, 21, 12, 24, 17, 16, 14, 13, 30, 18, and 25, and healthy subjects 104, 121, 24, 188, and 23 were present. FIGs. 27 and 28 are results of calculating a distance function (in this case, the magnitude of a deviation is indicated.) from a typical breast cancer patient pattern with high severity and a typical healthy subject pattern. From these results, of the cancer patients, while subjects 10, 7, 23, 19, 21, 12, 24, 17, 16, 14, 13, and 30 were determined to be "close to cancer", subjects 18 and 25 were determined to be "close to healthy" (FIG. 27). Furthermore, of the healthy subjects, subjects 121, 24, 188, 23, and 141 were determined to be "close to cancer", subjects 111, 28, and 131 were determined to be "close to healthy", and subject 104 was unable to be distinguished (FIG. 28).

The results of determination by the determination algorithm 2 (FIGs. 27 and 28) are:

$$\text{Sensitivity} = (28/30) \times 100 = 93.3\%,$$

$$\text{Specificity} = (204/210) \times 100 = 97.1\%.$$

However, it was determined that as for subjects 18 and 25 who are cancer patients determined to be close to healthy in the sensitivity, remeasurement is required for subject 18 because there was missing data, and subject 25 is under postoperative chemotherapy, thus a future evaluation is required. Therefore, by the present method, cancer was able to be evaluated with high accuracy in both sensitivity and specificity.

All publications, patents, and patent applications cited herein are hereby incorporated by reference in their entirety.

**Claims**

1. A method for testing cancer in a subject, the method comprising:

   preparing a database that has stored a marker panel on which information of multiple cancer markers with respect to multiple healthy subjects and cancer patients is registered, the database comprising discrimination information that classifies a measured value of each cancer marker, with a mean value of the healthy subjects $\pm$ X $\times$ standard deviation (wherein X is an arbitrary numerical value) and/or the mean value of the healthy subjects $\pm$ the standard deviation as a reference range, into any of three groups: within the reference range, higher than the reference range, and lower than the reference range;
   analyzing, with respect to measured values of one or more cancer markers of a subject, a correlation with the discrimination information in the database; and
   evaluating cancer in the subject on a basis of a result of the analyzing.

2. The method according to claim 1, wherein a marker panel of the subject is created on a basis of the measured values of the cancer markers of the subject.

3. The method according to claim 1, wherein, in the marker panel, columns of the cancer markers are displayed to be visually distinguishable according to three groups of the discrimination information.

4. The method according to claim 1, wherein, in the marker panel, the cancer markers are shown in order of importance calculated by machine learning.

5. The method according to claim 1, wherein, in the database, the reference range comprises a reference range of the mean value of the healthy subjects $\pm$ X $\times$ the standard deviation (wherein X is 2) and a reference range of the mean value of the healthy subjects $\pm$ the standard deviation, and a measured value of each cancer marker has different discrimination information for the two reference ranges.

6. The method according to claim 1, wherein, in accordance with three groups of the discrimination information, the measured values of the cancer markers are assigned a column value of 0 if it is within the reference range, a column value of + 1 if it is higher than the reference range, or a column value of -1 if it is lower than the reference range.

7. The method according to claim 6, wherein

the analyzing the correlation with the discrimination information is performed on a basis of a value of an evaluation function represented by Formula I:

$$\text{Evaluation function} = \sum_{n=1}^{m} \pm g_n \times \sqrt{(D_n^2)} \quad \text{(I)}$$

(wherein,
$g_n$ denotes a relative value of importance calculated by machine learning, and a sign of $g_n$ is + if a measured value of a cancer marker of the subject has a same column value as that of the cancer patients and - if the measured value has a different column value; and
$D_n$ denotes a column value of + 1, 0, or - 1).

8. The method according to claim 7, wherein the analyzing the correlation with the discrimination information is performed by calculating an evaluation function on a basis of the discrimination information with the mean value of the healthy subjects $\pm$ X $\times$ the standard deviation (wherein X is 2) as the reference range, and then calculating an evaluation function on a basis of the discrimination information with the mean value of the healthy subjects $\pm$ the standard deviation as the reference range.

9. The method according to claim 1 or 7, further comprising:

with respect to the measured values of the one or more cancer markers of the subject, calculating differences from a pattern of the discrimination information of the cancer patients and a pattern of the discrimination information of the healthy subjects in the database; and
analyzing which of the patterns of the cancer patients and the healthy subjects the measured values of the subject are close to.

10. The method according to claim 9, wherein the calculating the differences from the patterns is performed with a distance function represented by Formula II:

$$\text{Distance function} = \sqrt{\sum_{n=1}^{m} g_n^2 \times (D_n - T_n)^2} \quad \text{(II)}$$

(wherein,

$g_n$ denotes a relative value of importance calculated by machine learning;
$D_n$ denotes a row vector represented by a column value of + 1, 0, or -1 with respect to each cancer marker of the cancer patients or the healthy subjects in the database; and
$T_n$ denotes a row vector represented by a column value of + 1, 0, or - 1 with respect to each cancer marker in the subject).

11. The method according to claim 1, wherein the database comprises a marker panel on which information of multiple cancer markers with respect to a cancer patient in a specific stage or with a specific degree of severity is registered.

12. The method according to claim 1, wherein the cancer markers comprise 3 or more, 5 or more, 10 or more, or 20 or more cancer markers.

13. The method according to claim 1, wherein the cancer markers are urinary metabolites, and the marker panel on which the information of the cancer markers is registered is a metabolite list on which information of ion intensities of metabolites obtained by liquid chromatography mass spectrometry (LC/MS) is registered.

14. The method according to claim 1, wherein the database comprises different databases according to types of cancers.

15. The method according to claim 1, wherein the evaluating cancer comprises determination of cancer in the subject, prediction of a risk of cancer in the subject, determination of a stage or severity of cancer in the subject, prognostication of cancer in the subject, monitoring of cancer in the subject, monitoring of efficacy in the treatment of cancer present in the subject, or aid in diagnosis of cancer.

16. A system for testing cancer comprising:

a storage unit comprising a database that has stored a marker panel on which information of multiple cancer markers with respect to multiple healthy subjects and cancer patients is registered, the database comprising discrimination information that classifies a measured value of each cancer marker, with a mean value of the healthy subjects $\pm$ X $\times$ standard deviation (wherein X is an arbitrary numerical value) and/or the mean value of the healthy subjects $\pm$ the standard deviation as a reference range, into any of three groups: within the reference range, higher than the reference range, and lower than the reference range;
an input unit that is configured to receive inputs of measured values of one or more cancer markers of a subject;
an analysis unit that is configured to analyze, with respect to the measured values of the one or more cancer markers of the subject from the input unit, a correlation with the discrimination information in the database; and
an evaluation unit that is configured to evaluate cancer in the subject on a basis of an analysis result obtained by the analysis unit.

17. The system according to claim 16, wherein the system is configured to implement the method according to claim 1.

*FIG. 1*

PREDICTIVE VALUE

SUBJECT NUMBER

# FIG. 2

MARKER PANEL (IN ORDER OF IMPORTANCE IN MACHINE LEARNING)

| | | MARKERS | | | | |
|---|---|---|---|---|---|---|
| | | 1 | 2 | 3 | 4 | 5 |
| SUBJECTS | 1 | ION INTENSITY | ION INTENSITY | ION INTENSITY | ION INTENSITY | ION INTENSITY |
| | 2 | ION INTENSITY | ION INTENSITY | ION INTENSITY | ION INTENSITY | ION INTENSITY |
| | 3 | ION INTENSITY | ION INTENSITY | ION INTENSITY | ION INTENSITY | ION INTENSITY |
| | 4 | ION INTENSITY | ION INTENSITY | ION INTENSITY | ION INTENSITY | ION INTENSITY |
| | 5 | ION INTENSITY | ION INTENSITY | ION INTENSITY | ION INTENSITY | ION INTENSITY |

CONVERSION

| | | MARKERS | | | | |
|---|---|---|---|---|---|---|
| | | 1 | 2 | 3 | 4 | 5 |
| SUBJECTS | 1 | | | | | |
| | 2 | | | | | |
| | 3 | | | | | |
| | 4 | | | | | |
| | 5 | | | | | |

REFERENCE RANGE OF HEALTHY SUBJECTS

95%

2.5%                    2.5%

MEAN VALUE

$2\hat{\sigma}$ MODE 95.44%

$1\hat{\sigma}$ MODE 68.26%

EP 4 212 864 A1

19

## FIG. 3

## FIG. 4

# FIG. 5

$$\text{EVALUATION FUNCTION} = \sum_{n=1}^{m} \pm g_n \times \sqrt{D_n{}^2}$$

$g_n$ RELATIVE VALUE OF IMPORTANCE (MeanDecreaceAccuracy) OBTAINED BY RF METHOD
SIGN IS + IN A CASE WHERE COLUMN VALUE OF SUBJECT IS SAME VALUE AS THAT OF
CANCER PATIENT AND − IN A CASE WHERE IT IS A DIFFERENT COLUMN VALUE.

$D_n$ COLUMN VALUE: BLACK +1, WHITE 0, GRAY −1

| | | MARKERS | | | | |
|---|---|---|---|---|---|---|
| | | A | B | C | D | E |
| SUBJECTS | A | +1 | +1 | 0 | 0 | −1 |
| | B | +1 | +1 | 0 | 0 | −1 |
| | C | +1 | 0 | 0 | 0 | −1 |
| | D | +1 | 0 | 0 | +1 | −1 |
| | E | −1 | 0 | 0 | 0 | 0 |

# FIG. 6

$$\text{DISTANCE FUNCTION} = \sqrt{\sum_{n=1}^{m} g_n{}^2 \times (D_n - T_n)^2}$$

$$= \sqrt{\begin{aligned} & g_1{}^2 \times (\text{CANCER PATIENT·MARKER 1 COLUMN VALUE} - \text{SUBJECT 1·MARKER 1 COLUMN VALUE})^2 + \\ & g_2{}^2 \times (\text{CANCER PATIENT·MARKER 2 COLUMN VALUE} - \text{SUBJECT 1·MARKER 2 COLUMN VALUE})^2 + \end{aligned}} \quad \cdots$$

$g_n$  RELATIVE VALUE OF IMPORTANCE (MeanDecreaceAccuracy) OBTAINED BY RF METHOD

$D_n$  ROW VECTOR INDICATING VALUE OF COLUMN OF EACH MARKER OF CANCER AND HEALTHY SUBJECTS IN DATABASE (BLACK +1, WHITE 0, GRAY −1)

$T_n$  ROW VECTOR INDICATING VALUE OF COLUMN OF EACH MARKER FOR EACH SUBJECT (BLACK +1, WHITE 0, GRAY −1)

| | | MARKERS | | | | |
|---|---|---|---|---|---|---|
| | | 1 | 2 | 3 | 4 | 5 |
| $D_n$ | CANCER PATIENTS | +1 | +1 | +1 | −1 | −1 |
| | HEALTHY SUBJECTS | 0 | 0 | 0 | 0 | 0 |

| | | 1 | 2 | 3 | 4 | 5 |
|---|---|---|---|---|---|---|
| $T_n$ | 1 | +1 | 0 | 0 | 0 | −1 |
| | 2 | +1 | 0 | 0 | +1 | −1 |
| | 3 | −1 | 0 | 0 | 0 | 0 |

# FIG. 7

```
                                        ┌─────────────────────┐
                                        │ CREATE MARKER PANEL │
                                        └─────────────────────┘
                                                  │
        ┌──────────────────────────────────────────────────┐
        │ CALCULATE VALUE OF EVALUATION FUNCTION (2σ̂ MODE) │
        └──────────────────────────────────────────────────┘
                                │
            ┌───────────────────────────────┐
            │    PLOT EVALUATION FUNCTION    │
            └───────────────────────────────┘
```

α ≥ EVALUATION FUNCTION VALUE ≥ β (2σ̂ MODE)   α AND β ARE CONSTANTS

No → EVALUATION FUNCTION VALUE < β (2σ̂ MODE)   β IS CONSTANT

yes → HEALTHY

No → EVALUATION FUNCTION VALUE > α (2σ̂ MODE)   α IS CONSTANT

yes → CANCER

yes → EVALUATE EVALUATION FUNCTION VALUE OF CORRESPONDING SUBJECT (1σ̂ MODE)

EVALUATION FUNCTION VALUE < γ (1σ̂ MODE)   γ IS CONSTANT

yes → CLOSE TO HEALTHY

No → EVALUATION FUNCTION VALUE ≥ γ (1σ̂ MODE)   γ IS CONSTANT

yes → CLOSE TO CANCER

# FIG. 8

CREATE MARKER PANEL

⟨ (1) PART OF EVALUATION BY EVALUATION FUNCTION ⟩

CALCULATE VALUE OF EVALUATION FUNCTION

$\alpha$ > VALUE OF EVALUATION FUNCTION

$\alpha$ IS CONSTANT

No

VALUE OF EVALUATION FUNCTION > $\beta$

$\beta$ IS CONSTANT

yes

yes

LOW CANCER RISK (HEALTHY)

HIGH CANCER RISK (CANCER)

No

⟨ (2) PART OF EVALUATION BY DISTANCE FUNCTION ⟩

CALCULATE PATTERN OF $\beta \geq$ VALUE OF EVALUATION FUNCTION $\geq \alpha$ ($1\hat{\sigma}$ AND $2\hat{\sigma}$ MODES)

CALCULATE DISTANCE FUNCTION FROM TYPICAL CANCER PATIENT PATTERN AND HEALTHY SUBJECT PATTERN

CLOSE TO CANCER PATTERN?

No

yes

CLOSE TO CANCER

CLOSE TO HEALTHY

EP 4 212 864 A1

## FIG. 9

| METABOLITE | 1 | 2 | 3 | 4 | 5 | 6 | 7 | 8 | 9 | 10 |
|---|---|---|---|---|---|---|---|---|---|---|
| NAME OF COMPOUND | glycocholenate sulfate | glucuronide of C10H18O2 | X - A | N-methylproline | X - B | X - C | X - D | orotidine | X - E | 3-methylhistidine |
| SUPERPATHWAY | Lipid | Partially Characterized Molecules | - | Amino Acid | - | - | - | Nucleotide | - | Amino Acid |
| SUBPATHWAY | Secondary Bile Acid Metabolism | Partially Characterized Molecules | - | Urea cycle; Arginine and Proline Metabolism | - | - | - | Pyrimidine Metabolism, Orotate containing | - | Histidine Metabolism |

| METABOLITE | 11 | 12 | 13 | 14 | 15 | 16 | 17 |
|---|---|---|---|---|---|---|---|
| NAME OF COMPOUND | N-carbamoylalanine | X - F | glutamine conjugate of C8H12O4 | X - G | N-methyl-GABA | 2,3-dimethylsuccinate | galactosyl glycerol |
| SUPERPATHWAY | Amino Acid | - | Partially Characterized Molecules | - | Amino Acid | Amino Acid | Lipid |
| SUBPATHWAY | Alanine and Aspartate Metabolism | - | Partially Characterized Molecules | - | Glutamate Metabolism | Leucine, Isoleucine and Valine Metabolism | Glycolipid Metabolism |

EP 4 212 864 A1

FIG. 10

# FIG. 11

*FIG. 12*

CREATE METABOLITE LIST

CALCULATE VALUE OF EVALUATION FUNCTION (2ô MODE)

PLOT EVALUATION FUNCTION

22 ≥ EVALUATION FUNCTION VALUE ≥ 10 (2ô MODE)

α AND β ARE CONSTANTS

No → EVALUATION FUNCTION VALUE < 10 (2ô MODE)

No → EVALUATION FUNCTION VALUE > 22 (2ô MODE)

yes → HEALTHY

yes → CANCER

yes ↓

EVALUATE EVALUATION FUNCTION VALUE OF CORRESPONDING SUBJECT (1ô MODE)

EVALUATION FUNCTION VALUE < 50 (1ô MODE)

No → EVALUATION FUNCTION VALUE ≥ 50 (1ô MODE)

yes → CLOSE TO HEALTHY

yes → CLOSE TO CANCER

EP 4 212 864 A1

FIG. 13

## FIG. 14

| SUBJECT NUMBER | EVALUATION FUNCTION VALUE IN 2$\hat{\sigma}$ MODE | EVALUATION FUNCTION VALUE IN 1$\hat{\sigma}$ MODE | DETERMINATION RESULT |
|---|---|---|---|
| 1  2  3  4  5  6  7  8  9  10<br>12  13  14  15  16  19  20<br>21  22  23  24  25  28  29<br>30 | >22 | >85 | DETERMINED TO BE "CANCER" |
| 17 | 19.50 | 76.80 | DETERMINED TO BE "CLOSE TO CANCER" |
| 26 | 17.48 | 82.20 | DETERMINED TO BE "CLOSE TO CANCER" |
| 27 | 17.48 | 83.41 | DETERMINED TO BE "CLOSE TO CANCER" |
| 18 | 13.32 | 58.82 | DETERMINED TO BE "CLOSE TO CANCER" |
| 11 | 10.07 | 67.74 | DETERMINED TO BE "CLOSE TO CANCER" |
| 60 | 21.43 | 34.53 | DETERMINED TO BE "CLOSE TO HEALTHY" |
| 51 | 18.85 | 35.75 | DETERMINED TO BE "CLOSE TO HEALTHY" |
| 36 | 13.67 | 18.65 | DETERMINED TO BE "CLOSE TO HEALTHY" |
| 31  32  33  34  35  37  38<br>39  40  41  42  43  44  45<br>46  47  48  49  50  52  53<br>54  55  56  57  58  59 | <10 | <30 | DETERMINED TO BE "HEALTHY" |

DETERMINATION LINE THAT DISTINGUISHES BETWEEN CANCER AND HEALTHY

EP 4 212 864 A1

FIG. 15

CREATE METABOLITE LIST

CALCULATE VALUE OF EVALUATION FUNCTION

10 > VALUE OF EVALUATION FUNCTION

yes → LOW CANCER RISK (HEALTHY)

No ↓

VALUE OF EVALUATION FUNCTION > 22

yes → HIGH CANCER RISK (CANCER)

No ↓

CALCULATE PATTERN OF 22 ≥ VALUE OF EVALUATION FUNCTION ≥ 10 (1σ AND 2σ MODES)

CALCULATE DISTANCE FUNCTION FROM TYPICAL CANCER PATIENT PATTERN AND HEALTHY SUBJECT PATTERN

CLOSE TO CANCER PATTERN?

No → CLOSE TO HEALTHY

yes → CLOSE TO CANCER

# FIG. 16

# FIG. 17

● TYPICAL CANCER PATIENT PATTERN

● TYPICAL HEALTHY SUBJECT PATTERN

| | SUBJECT NUMBER | DISTANCE FROM HEALTHY SUBJECT 46 | DISTANCE FROM CANCER PATIENT 4 | DETERMINATION |
|---|---|---|---|---|
| 2σ̂ MODE | 4 | 17.82 | 0.00 | SUBJECT NUMBER 4 DETERMINED TO BE "CANCER PATIENT" |
| | 46 | 0.00 | 17.82 | |
| 1σ̂ MODE | 4 | 19.08 | 0.00 | SUBJECT NUMBER 46 DETERMINED TO BE "HEALTHY SUBJECT" |
| | 46 | 0.00 | 19.08 | |
| 2σ̂ MODE | 18 | 6.82 | 16.47 | SUBJECT NUMBER 18 DETERMINED TO BE "CLOSE TO CANCER" |
| | 28 | 6.82 | 16.47 | |
| | 51 | 6.82 | 16.47 | SUBJECT NUMBER 28 DETERMINED TO BE "CLOSE TO CANCER" |
| | 50 | 3.77 | 17.42 | |
| 1σ̂ MODE | 18 | 14.71 | 12.15 | SUBJECT NUMBER 51 DETERMINED TO BE "HEALTHY SUBJECT" |
| | 28 | 15.16 | 11.59 | |
| | 51 | 10.33 | 16.05 | SUBJECT NUMBER 50 DETERMINED TO BE "HEALTHY SUBJECT" |
| | 50 | 12.92 | 14.04 | |

EP 4 212 864 A1

FIG. 18

EP 4 212 864 A1

# FIG. 19

## FIG. 20

| METABOLITE | 1 | 2 | 3 | 4 | 5 | 6 | 7 | 8 |
|---|---|---|---|---|---|---|---|---|
| NAME OF COMPOUND | 5-hydroxymethyl-2-furoic acid | X - A | X - B | hypoxanthine | 1-methylnicotinamide | cis-urocanate | phosphate | 3-phosphoglycerate |
| SUPERPATHWAY | Amino Acid | - | - | Nucleotide | Cofactors and Vitamins | Amino Acid | Energy | Carbohydrate |
| SUBPATHWAY | Tyrosine Metabolism | - | - | Purine Metabolism, (Hypo)Xanthine/Inosine containing | Nicotinate and Nicotinamide Metabolism | Histidine Metabolism | Oxidative Phosphorylation | Glycolysis, Gluconeogenesis, and Pyruvate Metabolism |

| METABOLITE | 9 | 10 | 11 | 12 | 13 | 14 | 15 |
|---|---|---|---|---|---|---|---|
| NAME OF COMPOUND | uracil | X - C | X - D | ornithine | N-acetylalanine | glucuronide of C12H22O4 (2)* | palmitoyl sphingomyelin (d18:1/16:0) |
| SUPERPATHWAY | Nucleotide | - | - | Amino Acid | Amino Acid | Partially Characterized Molecules | Lipid |
| SUBPATHWAY | Pyrimidine Metabolism, Uracil containing | - | - | Urea cycle; Arginine and Proline Metabolism | Alanine and Aspartate Metabolism | Partially Characterized Molecules | Sphingomyelins |

FIG. 21

EP 4 212 864 A1

# FIG. 22

# FIG. 23

```
                                          ┌─────────────────────────┐
                                          │  CREATE METABOLITE LIST │
                                          └─────────────────────────┘
                                                     │
        ┌──────────────────────────────────────────┘
        ▼
┌────────────────────────────────────────────┐
│ CALCULATE VALUE OF EVALUATION FUNCTION (2σ̂ MODE) │
└────────────────────────────────────────────┘
        │
        ▼
┌────────────────────────────┐
│   PLOT EVALUATION FUNCTION  │
└────────────────────────────┘
        │
        ▼
```

40 ≥ EVALUATION FUNCTION VALUE ≥ 20 (2σ̂ MODE)
α AND β ARE CONSTANTS

No → EVALUATION FUNCTION VALUE < 20 (2σ̂ MODE)

No → EVALUATION FUNCTION VALUE > 40 (2σ̂ MODE)

yes → HEALTHY

yes → CANCER

yes ↓

EVALUATE EVALUATION FUNCTION VALUE OF CORRESPONDING SUBJECT (1σ̂ MODE)

→ EVALUATION FUNCTION VALUE < 40 (1σ̂ MODE)

No → EVALUATION FUNCTION VALUE ≥ 40 (1σ̂ MODE)

yes → CLOSE TO HEALTHY

yes → CLOSE TO CANCER

# FIG. 24

# FIG. 25

| SUBJECT NUMBER | EVALUATION FUNCTION VALUE IN $2\hat{\sigma}$ MODE |
|---|---|
| CANCER 10 | 41.35 |
| CANCER 7 | 39.548 |
| CANCER 23 | 39.253 |
| CANCER 19 | 36.052 |
| CANCER 21 | 35.469 |
| CANCER 12 | 35.414 |
| CANCER 24 | 35.064 |
| CANCER 17 | 34.881 |
| CANCER 16 | 32.235 |
| CANCER 14 | 32.154 |
| CANCER 13 | 29.759 |
| CANCER 30 | 26.711 |
| CANCER 18 | 21.222 |
| CANCER 25 | 18.981 |
| HEALTHY 104 | 33.783 |
| HEALTHY 121 | 31.802 |
| HEALTHY 24 | 31.72 |
| HEALTHY 188 | 29.999 |
| HEALTHY 23 | 25.555 |

| SUBJECT NUMBER | EVALUATION FUNCTION VALUE IN $1\hat{\sigma}$ MODE | DETERMINATION |
|---|---|---|
| CANCER 17 | 57.488 | DETERMINED TO BE "CLOSE TO CANCER" |
| CANCER 21 | 54.726 | DETERMINED TO BE "CLOSE TO CANCER" |
| CANCER 13 | 50.797 | DETERMINED TO BE "CLOSE TO CANCER" |
| HEALTHY 121 | 48.895 | DETERMINED TO BE "CLOSE TO CANCER" |
| CANCER 12 | 48.664 | DETERMINED TO BE "CLOSE TO CANCER" |
| CANCER 24 | 46.318 | DETERMINED TO BE "CLOSE TO CANCER" |
| CANCER 14 | 45.502 | DETERMINED TO BE "CLOSE TO CANCER" |
| CANCER 30 | 43.453 | DETERMINED TO BE "CLOSE TO CANCER" |
| HEALTHY 188 | 42.913 | DETERMINED TO BE "CLOSE TO CANCER" |
| CANCER 16 | 42.235 | DETERMINED TO BE "CLOSE TO CANCER" |
| HEALTHY 141 | 35.855 | DETERMINED TO BE "CLOSE TO HEALTHY" |
| HEALTHY 24 | 35.611 | DETERMINED TO BE "CLOSE TO HEALTHY" |
| CANCER 18 | 34.566 | DETERMINED TO BE "CLOSE TO HEALTHY" |
| CANCER 25 | 34.092 | DETERMINED TO BE "CLOSE TO HEALTHY" |
| HEALTHY 104 | 30.693 | DETERMINED TO BE "CLOSE TO HEALTHY" |
| HEALTHY 23 | 26.576 | DETERMINED TO BE "CLOSE TO HEALTHY" |
| HEALTHY 28 | 24.198 | DETERMINED TO BE "CLOSE TO HEALTHY" |
| HEALTHY 111 | 24.193 | DETERMINED TO BE "CLOSE TO HEALTHY" |
| HEALTHY 131 | 23.934 | DETERMINED TO BE "CLOSE TO HEALTHY" |

DETERMINATION LINE THAT DISTINGUISHES BETWEEN CANCER AND HEALTHY

EP 4 212 864 A1

FIG. 26

CALCULATE PATTERN OF
40 ≥ VALUE OF EVALUATION FUNCTION ≥ 20
(1ô̂ AND 2ô̂ MODES)

CALCULATE DISTANCE FUNCTION FROM TYPICAL
CANCER PATIENT PATTERN AND HEALTHY SUBJECT
PATTERN

IS RATIO OF
DISTANCE FUNCTION VALUE IN 2ô̂ IS EQUAL
TO OR MORE THAN 1.2?

No

yes

ARE RESULTS OF
DETERMINATIONS IN TWO MODES
SAME?

No

RATIO OF DISTANCE
FUNCTION VALUE IN 1ô̂
> 1.2?

No

yes

yes

DETERMINED TO BE CANCER?

No

DETERMINED TO BE
CANCER IN 1ô̂ MODE?

No

yes

yes

CLOSE TO CANCER

CLOSE TO HEALTHY

CLOSE TO CANCER

CLOSE TO HEALTHY

FAILED TO BE DETERMINED

EP 4 212 864 A1

## FIG. 27

● TYPICAL CANCER PATIENT PATTERN

● TYPICAL HEALTHY SUBJECT PATTERN

| | SUBJECT NUMBER | 2σ̂ MODE | | 1σ̂ MODE | | DETERMINATION RESULT |
|---|---|---|---|---|---|---|
| | | DISTANCE FROM HEALTHY SUBJECT PATTERN | DISTANCE FROM CANCER PATIENT PATTERN | DISTANCE FROM HEALTHY SUBJECT PATTERN | DISTANCE FROM CANCER PATIENT PATTERN | |
| CANCER PATIENTS | 2  3  4  5  6  8<br>9  11  15  20<br>22  26  27  28<br>29  31 | - | - | - | - | DETERMINED TO BE "CANCER" SINCE EVALUATION FUNCTION VALUE IN 2σ̂ MODE>40 |
| | 10 | 17.16 | 11.09 | 17.33 | 10.82 | DETERMINED TO BE "CLOSE TO CANCER" |
| | 7 | 16.67 | 9.73 | 17.34 | 8.49 | DETERMINED TO BE "CLOSE TO CANCER" |
| | 23 | 8.94 | 17.92 | 19.13 | 8.92 | DETERMINED TO BE "CLOSE TO CANCER" |
| | 19 | 14.19 | 13.08 | 17.34 | 8.49 | DETERMINED TO BE "CLOSE TO CANCER" |
| | 21 | 16.17 | 11.81 | 18.42 | 7.83 | DETERMINED TO BE "CLOSE TO CANCER" |
| | 12 | 13.94 | 14.37 | 17.46 | 9.79 | DETERMINED TO BE "CLOSE TO CANCER" |
| | 24 | 16.08 | 12.60 | 17.35 | 10.79 | DETERMINED TO BE "CLOSE TO CANCER" |
| | 17 | 14.04 | 17.68 | 18.34 | 13.16 | DETERMINED TO BE "CLOSE TO CANCER" |
| | 16 | 11.73 | 16.22 | 15.42 | 12.77 | DETERMINED TO BE "CLOSE TO CANCER" |
| | 14 | 15.78 | 11.12 | 17.15 | 8.87 | DETERMINED TO BE "CLOSE TO CANCER" |
| | 13 | 14.80 | 12.40 | 18.00 | 6.96 | DETERMINED TO BE "CLOSE TO CANCER" |
| | 30 | 14.28 | 12.99 | 16.59 | 12.87 | DETERMINED TO BE "CLOSE TO CANCER" |
| | 18 | 8.71 | 18.03 | 13.67 | 16.08 | DETERMINED TO BE "CLOSE TO HEALTHY" |
| | 25 | 6.78 | 19.33 | 14.19 | 14.78 | DETERMINED TO BE "CLOSE TO HEALTHY" |

EP 4 212 864 A1

## FIG. 28

| | SUBJECT NUMBER | 2σ MODE | | 1σ MODE | | DETERMINATION RESULT |
|---|---|---|---|---|---|---|
| | | DISTANCE FROM HEALTHY SUBJECT PATTERN | DISTANCE FROM CANCER PATIENT PATTERN | DISTANCE FROM HEALTHY SUBJECT PATTERN | DISTANCE FROM CANCER PATIENT PATTERN | |
| HEALTHY SUBJECTS | 104 | 14.12 | 13.17 | 15.47 | 17.12 | FAILED TO DISTINGUISH BETWEEN CANCER AND HEALTHY SUBJECT |
| | 121 | 16.44 | 12.13 | 18.59 | 10.01 | DETERMINED TO BE "CLOSE TO CANCER" |
| | 24 | 15.73 | 11.19 | 16.20 | 10.49 | DETERMINED TO BE "CLOSE TO CANCER" |
| | 188 | 15.16 | 13.08 | 17.00 | 10.57 | DETERMINED TO BE "CLOSE TO CANCER" |
| | 23 | 15.11 | 12.01 | 17.20 | 15.04 | DETERMINED TO BE "CLOSE TO CANCER" |
| | 141 | 14.28 | 12.99 | 16.81 | 12.47 | DETERMINED TO BE "CLOSE TO CANCER" |
| | 111 | 12.31 | 14.87 | 14.88 | 16.02 | DETERMINED TO BE "CLOSE TO HEALTHY" |
| | 28 | 11.87 | 16.12 | 17.83 | 18.10 | DETERMINED TO BE "CLOSE TO HEALTHY" |
| | 131 | 11.98 | 15.14 | 16.55 | 14.84 | DETERMINED TO BE "CLOSE TO HEALTHY" |
| | . . . | . . . | . . . | . . . | . . . | DETERMINED TO BE "HEALTHY" SINCE EVALUATION FUNCTION VALUE IN 2σ MODE < 20 |

EP 4 212 864 A1

# FIG. 29

## INTERNATIONAL SEARCH REPORT

| | International application No. |
|---|---|
| | PCT/JP2020/034198 |

A. CLASSIFICATION OF SUBJECT MATTER
Int.Cl. G01N27/62(2021.01)i, G01N33/50(2006.01)i
FI: G01N33/50T, G01N27/62X, G01N33/50R

According to International Patent Classification (IPC) or to both national classification and IPC

B. FIELDS SEARCHED

Minimum documentation searched (classification system followed by classification symbols)
Int.Cl. G01N27/62, G01N33/50

Documentation searched other than minimum documentation to the extent that such documents are included in the fields searched
Published examined utility model applications of Japan       1922-1996
Published unexamined utility model applications of Japan       1971-2020
Registered utility model specifications of Japan       1996-2020
Published registered utility model applications of Japan       1994-2020

Electronic data base consulted during the international search (name of data base and, where practicable, search terms used)
JSTPlus/JMEDPlus/JST7580(JDreamIII)

C. DOCUMENTS CONSIDERED TO BE RELEVANT

| Category* | Citation of document, with indication, where appropriate, of the relevant passages | Relevant to claim No. |
|---|---|---|
| A | JP 2019-168319 A (NAGOYA UNIVERSITY) 03 October 2019 (2019-10-03), entire text, all drawings | 1-17 |
| A | JP 2011-527414 A (CELERA CORPORATION) 27 October 2011 (2011-10-27), entire text, all drawings | 1-17 |
| A | WO 2017/165956 A1 (UTI LIMITED PARTNERSHIP) 05 October 2017 (2017-10-05), entire text, all drawings | 1-17 |
| A | WO 2017/213246 A1 (HITACHI, LTD.) 14 December 2017 (2017-12-14), entire text, all drawings | 1-17 |
| A | JP 2010-532480 A (ABBOTT LABORATORIES) 07 October 2010 (2010-10-07), entire text, all drawings | 1-17 |

☐ Further documents are listed in the continuation of Box C.       ☒ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| 26 November 2020 | 08 December 2020 |

| Name and mailing address of the ISA/ | Authorized officer |
|---|---|
| Japan Patent Office 3-4-3, Kasumigaseki, Chiyoda-ku, Tokyo 100-8915, Japan | Telephone No. |

Form PCT/ISA/210 (second sheet) (January 2015)

## INTERNATIONAL SEARCH REPORT
Information on patent family members

| International application No. |
|---|
| PCT/JP2020/034198 |

| | | |
|---|---|---|
| JP 2019-168319 A | 03 October 2019 | US 2019/0293653 A1 entire text, all drawings |
| JP 2011-527414 A | 27 October 2011 | US 2012/0076725 A1 entire text, all drawings WO 2009/067546 A2 EP 2227556 A2 |
| WO 2017/165956 A1 | 05 October 2017 | (Family: none) |
| WO 2017/213246 A1 | 14 December 2017 | US 2019/0094205 A1 entire text, all drawings CN 108603859 A JP 6692422 B2 |
| JP 2010-532480 A | 07 October 2010 | US 2008/0160546 A1 entire text, all drawings WO 2009/006323 A2 EP 2171464 A2 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2019105456 A
- JP 2020079729 A
- WO 2007076439 A
- WO 2011119772 A
- US 20090004687 A1
- WO 2017213246 A
- JP 2019168319 A